# EUROPEAN PATENT APPLICATION

(11) **EP 4 484 425 A1**
(43) Date of publication of application: **01.01.2025**
(21) Application number: 23760032.5
(22) Date of filing: 22.02.2023
(51) Int. Cl.: C07D 417/06, A61K 31/4427, A61K 31/497, A61K 51/00, A61P 25/16, A61P 25/28, A61P 43/00

(54) **NOVEL COMPOUND, ALPHA-SYNUCLEIN AGGREGATE BINDER, AND USE THEREOF**

(30) Priority: 24.02.2022 JP 2022027279
(71) Applicant: National Institutes for Quantum Science and Technology, Chiba 263-8555 (JP); Eisai R&D Management Co., Ltd., Tokyo 112-8088 (JP); ONO Pharmaceutical Co., Ltd., Osaka-shi, Osaka 541-8526 (JP); Takeda Pharmaceutical Company Limited, Osaka-shi, Osaka 541-0045 (JP)
(72) Inventor: HIGUCHI, Makoto, Chiba-shi, Chiba 263-8555 (JP); ONO, Maiko, Chiba-shi, Chiba 263-8555 (JP); CHO, Meiei, Chiba-shi, Chiba 263-8555 (JP); TAKADO, Yuhei, Chiba-shi, Chiba 263-8555 (JP); MATSUOKA, Kiwamu, Chiba-shi, Chiba 263-8555 (JP); MIZUMA, Hiroshi, Chiba-shi, Chiba 263-8555 (JP); YAMAMOTO, Takeshi, Fujisawa-shi, Kanagawa 251-0012 (JP); WAKABAYASHI, Takeshi, Fujisawa-shi, Kanagawa 251-0012 (JP); OHFUSA, Toshiyuki, Tsukuba-shi, Ibaraki 300-2635 (JP)
(74) Representative: Plasseraud IP
(86) International application number: PCT/JP2023/006432
(87) International publication number: WO 2023/163033

(57) **Abstract**

Provided is a compound that has high binding selectivity with respect to α-synuclein aggregates.

Provided is a compound represented by the following formula (I), (II), or (III), a pharmaceutically acceptable salt thereof, or a solvate thereof.

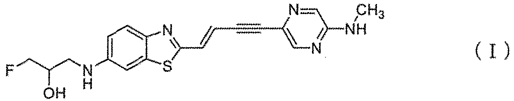

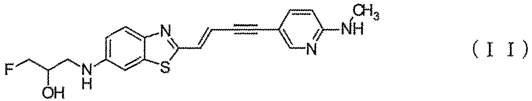

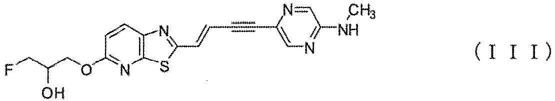

## Description

### Technical Field

The present invention relates to a novel compound, an α-synuclein aggregate binding agent, and use thereof, specifically, relates to a novel compound, an α-synuclein aggregate binding agent containing the novel compound, a composition for optical imaging of α-synuclein aggregates, a composition for radiological imaging of α-synuclein aggregates, a method for carrying out optical imaging of α-synuclein aggregates in brain, a method for carrying out radiological imaging of α-synuclein aggregates in brain, and an intermediate for synthesizing the novel compound.

### Background Art

α-synuclein is a protein that localizes at a neuronal synapse and the like. It has been considered that aggregates of α-synuclein (hereinafter referred to as α-synuclein aggregates) form a core pathology of Parkinson's disease, dementia with Lewy bodies (DLB), and multiple system atrophy (MSA) and have a close causal association with neurodegeneration. Definitive diagnoses of these diseases are carried out while the presence of α-synuclein aggregates (also referred to as "a-synuclein lesion" herein) is used as an indicator in a pathological examination of the autopsied brain, and it is therefore impossible to make a definitive diagnosis while a patient is alive. However, if α-synuclein aggregates can be visualized in the brain of the living patient, it is possible to obtain information close to definitive diagnoses of these diseases from an early stage. Moreover, if α-synuclein aggregates can be visualized in the brain of a living disease model animal, such visualization can also help assess the efficacy of a candidate substance for a therapeutic or preventive agent targeting the α-synuclein aggregates, by imaging over time and the like.

[¹¹C]BF-227 is an example of a positron emission tomography (PET) probe that has previously shown to bind to α-synuclein in the brain (Non-Patent Literatures 1 and 2). However, [¹¹C]BF-227 has insufficient binding affinity with respect to the α-synuclein aggregates, and α-synuclein lesions can be detected only in a subset of patients with MSA among the above diseases. In addition, [¹¹C]BF-227 has a problem of nonspecific accumulation in the brain and a problem of low binding selectivity with respect to the α-synuclein aggregates because [¹¹C]BF-227 binds to amyloid-β aggregates.

The inventors developed a compound for imaging a tau protein accumulated in the brain (see Patent Literature 1). The compound described in Patent Literature 1 allows imaging of a tau protein accumulated in the brain, and therefore the technology of Patent Literature 1 is useful for treatment, prevention, and the like of a disease (e.g., Alzheimer's disease (AD)) caused by accumulation of a tau protein. However, Patent Literature 1 does not describe the compound's binding to the α-synuclein aggregates.

### Citation List

### [Patent Literatures]

[Patent Literature 1]
   International Publication No. WO 2014/097474
[Patent Literature 2]
   International Publication No. WO 2020/174963

### [Non-patent Literatures]

[Non-patent Literature 1]
   Kikuchi, A. et al., Brain, 133:1772-1778 (2010)
[Non-patent Literature 2]
   Verdurand, M. et al., Contrast Media Mol. Imaging, 2018: 9165458 (2018)

### Summary of Invention

### Technical Problem

The present invention has been accomplished in view of the above circumstances, and its objective is to provide an α-synuclein aggregate binding agent having high binding selectivity with respect to α-synuclein aggregates, a method for carrying out imaging with use of the α-synuclein aggregate binding agent, and a novel compound which can be used for an α-synuclein aggregate binding agent.

### Solution to Problem

The inventors of the present invention found that a compound having a specific structure has high binding selectivity with respect to α-synuclein aggregates, and conducted further studies to complete the present invention. More specifically, the present invention provides the following features.

An aspect of the present invention is a compound represented by the following formula (I), (II), or (III), a pharmaceutically acceptable salt thereof, or a solvate thereof.

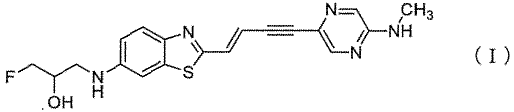

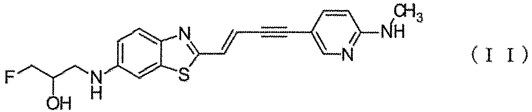

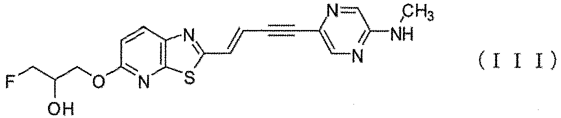

### Advantageous Effects of Invention

According to the present invention, it is possible to provide an α-synuclein aggregate binding agent having high binding selectivity with respect to α-synuclein aggregates.

### Brief Description of Drawings

Fig. 1 is a diagram showing results of fluorescence microscope measurement of the brains of DLB and AD patients.
Fig. 2 is a diagram showing results of quantifying fluorescence in lesion-enriched areas and lesion-free areas.
Fig. 3 is a diagram showing results of fluorescence microscope measurement of an α-synuclein fibril-inoculated mouse.
Fig. 4 is a diagram showing results of two-photon laser scanning fluorescence microscopy of an α-synuclein fibril-inoculated mouse.

### Description of Embodiments

The following description describes embodiments of the present invention. Note that, as used herein, the expression "A and/or B" is intended to mean at least one of A and B.

### [Definition]

The term "pharmaceutically acceptable salt" refers to a salt that is not harmful to mammals, particularly to humans. The pharmaceutically acceptable salt can be formed with use of a non-toxic acid or base. The non-toxic acid includes inorganic acids and organic acids, and the non-toxic base includes inorganic bases and organic bases. Examples of the pharmaceutically acceptable salt include: metal salts formed from aluminum, calcium, lithium, magnesium, potassium, sodium, zinc, and the like; and organic salts formed from lysine, N,N'-dibenzylethylenediamine, chloroprocaine, choline, diethanolamine, ethylenediamine, meglumine (N-methylglucamine), procaine, and the like. The pharmaceutically acceptable salt also includes acid-addition salts and base-addition salts.

The term "pharmaceutically acceptable carrier" means a pharmaceutically acceptable material, composition, or vehicle, such as a physiological saline solution, a liquid or solid filler, a diluent, a solvent, or an encapsulant. Examples of the pharmaceutically acceptable carrier include water, saline, physiological saline, phosphate buffered saline (PBS), sodium chloride injection solutions, Ringer's injection solutions, isotonic dextrose injection solutions, sterile water injection solutions, dextrose, and lactated Ringer's injection solutions.

The term "effective amount" refers to the amount of a compound or a composition which amount makes it possible to bring about an intended effect. For example, in some aspects, the effective amount refers to the amount of a compound or a composition which amount enables optical or radiological imaging of a substance (such as α-synuclein aggregates) accumulated in the brain.

The term "solvate" means a solvent-containing compound that is formed by association of one or more solvent molecules to a compound. Examples of the solvate include monosolvates, disolvates, trisolvates, and tetrasolvates. The solvate also includes hydrates.

The term "hydrate" means a compound that further contains a stoichiometric or a non-stoichiometric amount of water constrained by a non-covalent bonding intermolecular force, or a salt thereof. Examples of the hydrate include monohydrates, dihydrates, trihydrates, and tetrahydrates.

The term "treatment" means moderating or remitting progress, the severity, and/or a duration of a disease or condition.

The term "prevention" means reducing a risk of catching or progressing a given disease or condition, or reducing or inhibiting relapse, onset, or progress of one or more symptoms of a given disease or condition.

The term "binding performance" means strength with which a compound binds to specific protein aggregates.

The term "binding selectivity" indicates that, when compared, the binding performance of a compound with respect to specific protein aggregates and the binding performance of the compound with respect to other protein aggregates differ from each other (the binding performance with respect to the specific protein aggregates is higher or lower than the binding performance with respect to the other protein aggregates). The phrase "high binding selectivity" means that the above difference in binding performance is large. For example, that a compound has "high binding selectivity with respect to α-synuclein aggregates" indicates that there is a large difference between the binding performance of the compound with respect to the α-synuclein aggregates and the binding performance of the compound with respect to other protein aggregates and the compound has higher binding performance with respect to the α-synuclein aggregates.

### [Compound]

In an embodiment, the present invention provides (E)-1-fluoro-3-((2-(4-(5-(methylamino)pyrazin-2-yl)but-1-en-3-yn-1-yl)benzo[d]thiazol-6-yl)amino)propan-2-ol, which is represented by the following structural formula (I), (E)-1-fluoro-3-((2-(4-(6-(methylamino)pyridin-3-yl)but-1-en-3-yn-1-yl)benzo[d]thiazol-6-yl)amino)propan-2-ol, which is represented by the following structural formula (II), or (E)-1-fluoro-3-((2-(4-(5-(methylamino)pyrazin-2-yl)but-1-en-3-yn-1-yl)thiazolo[5,4-b]pyridin-5-yl)oxy)propan-2-ol, which is represented by the following structural formula (III), a pharmaceutically acceptable salt thereof, or a solvate thereof.

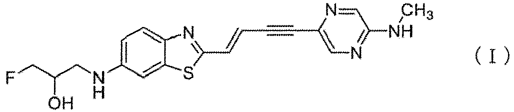

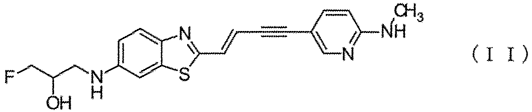

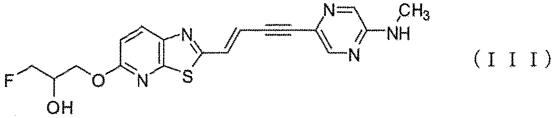

Herein, the compounds represented by formulae (I), (II), and (III) will be also referred to as "compound (I)", "compound (II)", and "compound (III)", respectively.

In an aspect, the compound (I), the compound (II), and the compound (III) are each a compound in which at least one atom is a radioisotope thereof, a salt thereof, or a solvate thereof. The radioisotope is selected from the group consisting of ¹⁵O, ¹³N, ¹¹C, ¹⁸F, and the like, but is not particularly limited. Preferably, the radioisotope is ¹¹C or ¹⁸F. Among these, in consideration of the fact that the half-life of ¹¹C is approximately 20 minutes and the half-life of ¹⁸F is approximately 110 minutes, a compound that is labeled with ¹⁸F would be commercially more useful. Therefore, the radioisotope is most preferably ¹⁸F.

Preferably, at least one of a methylamino group binding to a pyrazine ring or a pyridine ring and a 3-fluoro-2-hydroxypropylamino group (-NH-CH₂-CH(OH)-CH₂F) binding to a benzothiazole ring or a 3-fluoro-2-hydroxypropoxy group (-O-CH₂-CH(OH)-CH₂F) binding to a thiazolopyridine ring is a group containing a radioisotope. More preferably, the 3-fluoro-2-hydroxypropylamino group or the 3-fluoro-2-hydroxypropoxy group is a group containing a radioisotope. Still more preferably, a fluorine atom in the 3-fluoro-2-hydroxypropylamino group or the 3-fluoro-2-hydroxypropoxy group is a radioisotope.

In an aspect, the compound (I) which contains a radioisotope is preferably [¹⁸F]-(E)-1-fluoro-3-((2-(4-(5-(methylamino)pyrazin-2-yl)but-1-en-3-yn-1-yl)benzo[d]thiazol-6-yl)amino)propan-2-ol.

In an aspect, the compound (II) which contains a radioisotope is preferably [¹⁸F]-(E)-1-fluoro-3-((2-(4-(6-(methylamino) pyridin-3 -yl) bu t-1-en-3 -yn-1-yl)benzo[d]thiazol-6-yl)amino)propan-2-ol.

In an aspect, the compound (III) which contains a radioisotope is preferably [¹⁸F]-(E)-1-fluoro-3-((2-(4-(5-(methylamino)pyrazin-2-yl)but-1-en-3-yn-1-yl)thiazolo[5,4-b]pyridin-5-yl)oxy)propan-2-ol.

### [Intermediate]

As shown in a method for producing the compound below, a compound represented by the following formula (IV) is a production intermediate compound produced during production of the compound (I) and the compound (II). Further, a compound represented by the following formula (V) is a production intermediate compound produced during production of the compound (III). Further, a compound represented by the following formula (VI) is a production intermediate compound produced during production of the compound (I) and the compound (II). Herein, these production intermediate compounds are simply referred to as "intermediate compounds" or "intermediates".

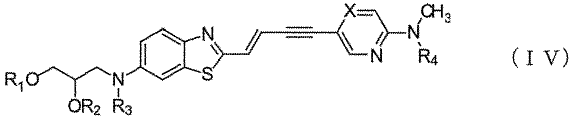

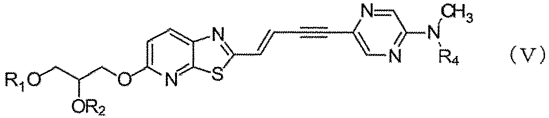

In the formula (IV) and the formula (V), R₁ is a 4-nitrobenzenesulfonyl group, a p-toluenesulfonyl group (tosyl group), or a methanesulfonyl group. R₂ is a tetrahydro-2H-pyran-2-yl group or a methoxymethyl group. R₄ is a hydrogen atom, a tert-butoxycarbonyl (Boc) group, or a 2,4-dimethoxybenzyl group.

In the formula (IV), R₃ is a hydrogen atom, a tert-butoxycarbonyl group, or a 2,4-dimethoxybenzyl group. X is a nitrogen atom or an unsubstituted carbon atom. The term "unsubstituted carbon atom" herein indicates CH. 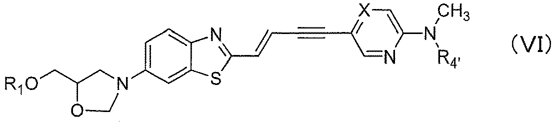 In the formula (VI),
R₁ is a 4-nitrobenzenesulfonyl group, a p-toluenesulfonyl group, or a methanesulfonyl group,
R₄ is a hydrogen atom or a tert-butoxycarbonyl group, and
X is a nitrogen atom (N) or an unsubstituted carbon atom (CH).

These intermediate compounds are suitably used to synthesize the compound (I) and the compound (II) or to synthesize the compound (III) and further to synthesize the compound (I) and the compound (II) each of which is labeled with a radioisotope or to synthesize the compound (III) which is labeled with a radioisotope. These intermediate compounds may be salts.

In a case where the compound (I), the compound (II), or the compound (III) has any of various isomers such as stereoisomers (including optical isomers and rotamers), tautomers, and polar isomers, such isomers are also included in the compound (I), the compound (II), or the compound (III). These isomers can be each obtained as a single isomer, by a known synthesizing method or a separating method. The compound (IV), the compound (V), and the compound (VI) can also each include various isomers.

The compounds (I) to (VI) may be each a crystal produced by a known crystallizing method.

### [Method for producing compounds]

The compounds (I) to (VI) can be produced in accordance with a method based on a production method below. As desired, the compounds (I) to (VI) can be produced by carrying out one or a combination of two or more of a deprotection reaction, an amidation reaction, a ureation reaction, an alkylation reaction, a Mitsunobu reaction, an oxidation reaction, a reduction reaction, a halogenation reaction, a coupling reaction, a nucleophilic addition reaction by a carbanion, a Grignard reaction, a dehydration reaction, and the like. Conditions, such as a solvent, a reagent, and a temperature, of each reaction can be set as appropriate, on the basis of the common technical knowledge of a person skilled in the art. A protection reaction and a deprotection reaction of a functional group are each carried out in accordance with a known reaction method or a method described in Reference Examples or Examples. As a protecting group, a conventional group is used.

Each symbol used in the following production method has the same meaning as the above-described symbol, unless otherwise specified.

Further, in the description of the following production methods, compounds represented by (1), (2), and the like in schemes indicated in the production methods will be referred to as a compound (1), a compound (2), and the like, respectively.

### [1. Method for producing compound (I) and compound (II) and compound (IV) which is intermediate of compound (I) and compound (II)]

### (Production Method A)

As an aspect, the compound (I) and the compound (II) can be produced by a method shown in Production Scheme 1 below.

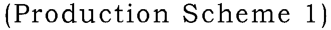

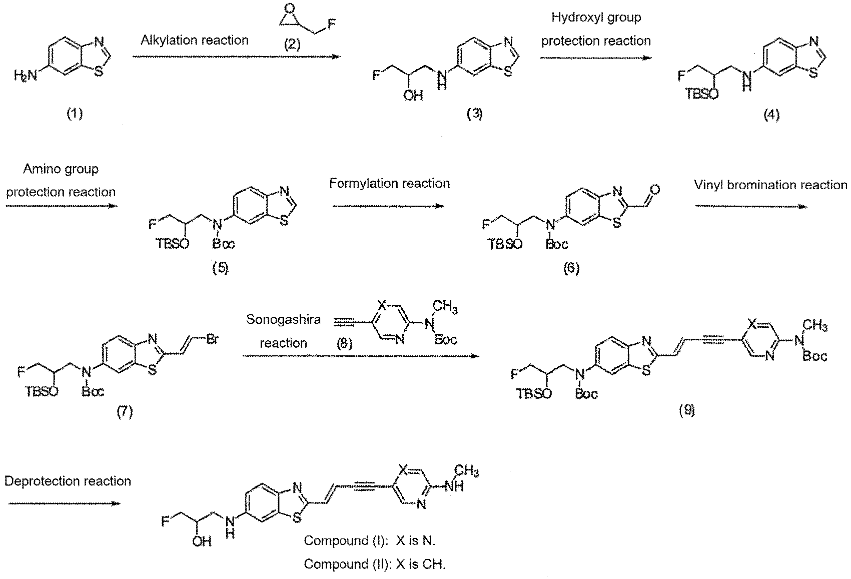

In Production Scheme 1, TBS represents a tertbutyldimethylsilyl group.

A compound (3) can be produced by an alkylation reaction of a compound (1) involving an oxirane ring-opening reaction of a compound (2).

A compound (4) can be produced by a hydroxyl group protection reaction of the compound (3) by tertbutyldimethylsilyl chloride.

A compound (5) can be produced by an amino group protection reaction of the compound (4) by di-tert-butyl dicarbonate.

A compound (6) can be produced by a formylation reaction of the compound (5) by N,N-dimethylformamide or the like.

A compound (7) can be produced by a vinyl bromination reaction of the compound (6) with use of (bromomethyl)triphenylphosphonium bromide.

A compound (9) can be produced by a Sonogashira reaction or the like between the compound (7) and a compound (8) (described later).

The compound (I) and the compound (II) can be produced by a deprotection reaction of the compound (9).

The compound (8) used in Production Method A can be produced from a compound (10) by a method shown in Production Scheme 2 below.

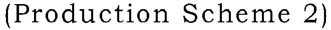

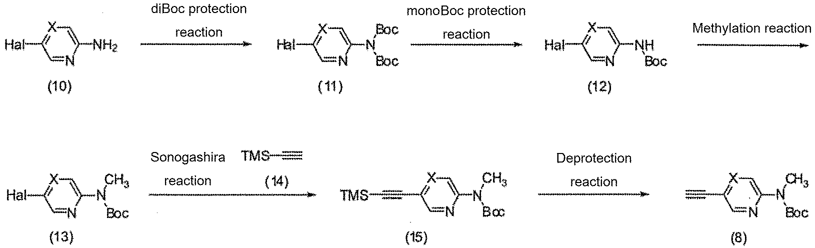

In Production Scheme 2, Hal represents a halogen atom (e.g., a bromine atom or an iodine atom), and TMS represents a trimethylsilyl group.

A compound (11) can be produced by an amino group protection (diBoc protection) reaction of the compound (10) by di-tert-butyl dicarbonate.

A compound (12) can be produced by deprotection reaction of one Boc group (monoBoc protection) in the compound (11) by a base such as potassium carbonate in a solvent such as methanol.

A compound (13) can be produced by a methylation reaction of the compound (12) by iodine methane or the like.

A compound (15) can be produced by a Sonogashira reaction between the compound (13) and ethynyltrimethylsilane (14).

A compound (8) can be produced by a deprotection reaction of a TMS group in the compound (15).

### (Production Method B)

As another aspect, the compound (I) and the compound (II) can also be produced by a method shown in Production Scheme 3 below.

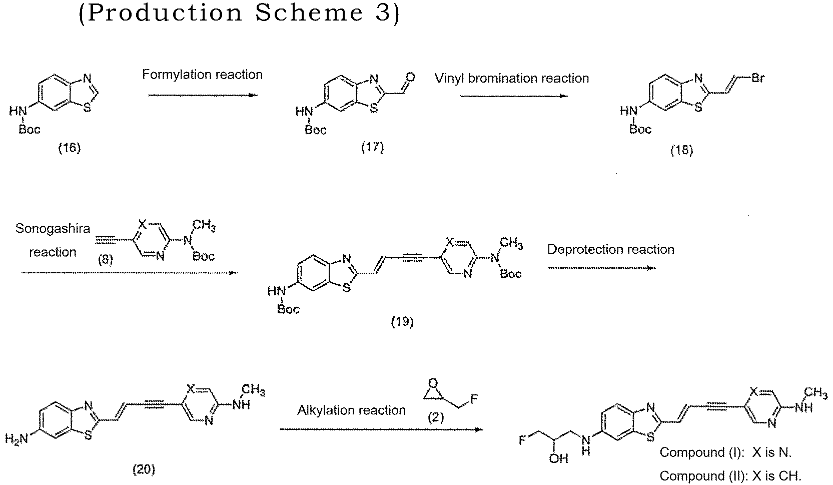

A compound (17) can be produced by a formylation reaction of the compound (16) by N,N-dimethylformamide or the like.

A compound (18) can be produced by a vinyl bromination reaction of the compound (17) with use of (bromomethyl)triphenylphosphonium bromide.

A compound (19) can be produced by a Sonogashira reaction or the like between the compound (18) and the compound (8).

A compound (20) can be produced by a deprotection reaction of the compound (19).

The compound (I) and the compound (II) can be each produced by an alkylation reaction of the compound (20) involving an oxirane ring-opening reaction of the compound (2).

In addition, when the compound (2) in which a fluorine atom is ¹⁸F, that is, [¹⁸F]2-(fluoromethyl)oxirane (CAS [123436-06-6]), is used in the alkylation reaction of the compound (20) involving an oxirane ring-opening reaction of the compound (2), the compounds (I) and (II) in which a fluorine atom in 3-fluoro-2-hydroxypropylamino group is a radioisotope can be produced. Here, [¹⁸F]2-(fluoromethyl)oxirane can be synthesized by (i) nucleophilic substitution of glycidyl tosylate with use of [¹⁸F]fluoride ions and (ii) purification by distillation. An example of synthesis of [¹⁸F]2-(fluoromethyl)oxirane is shown below.

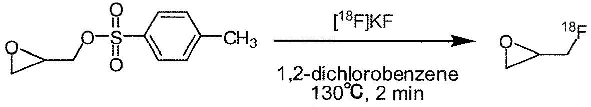

### (Production Method C)

As still another aspect, the compound (IV) in which R₃ and R₄ are each a Boc group (compound (IV-i)) can be produced by a method shown in Production Scheme 4 below.

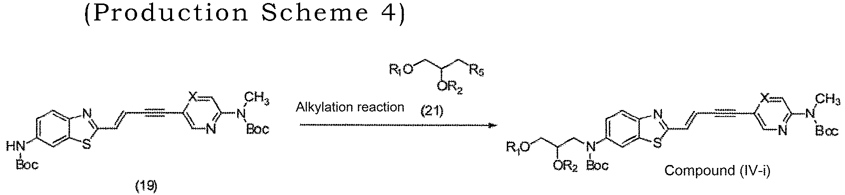

In Production Scheme 4, R₅ represents a halogen atom (e.g., a bromine atom or an iodine atom) or a leaving group such as a 4-nitrobenzenesulfonyloxy group.

A compound (IV-i) can be produced by an alkylation reaction of the compound (19) by a compound (21) (described later).

A compound (21a) in which, in the compound (21) used in Production Method C, R₅ is a halogen atom, and R₁ is a tosyl group or a 4-nitrobenzenesulfonyl group can be produced from a compound (22) by a method shown in Production Scheme 5 below.

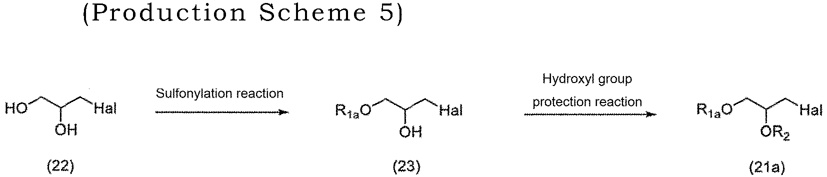

In Production Scheme 5, R₁ₐ represents a tosyl group or a 4-nitrobenzenesulfonyl group, and Hal represents a halogen atom (e.g., an iodine atom).

A compound (23) can be produced by a sulfonylation reaction of the compound (22).

The compound (21a) can be produced by a hydroxyl group protection reaction of the compound (23) by a tetrahydropyranyl (THP) group, a methoxymethyl (MOM) group, or the like.

### (Production Method C')

The compounds (I) and (II) can be each produced from a production intermediate compound (IV) by a method shown in Production Scheme 4' below.

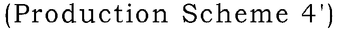

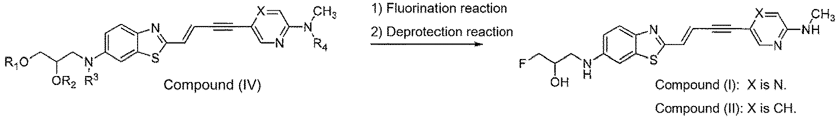

The compounds (I) and (II) can be each produced by a deprotection reaction after a fluorination reaction of the production intermediate compound (IV) by potassium fluoride or the like. Further, the compounds (I) and (II) which are radioisotopes can be produced with use of [¹⁸F]potassium fluoride or the like.

### [2. Method for producing compound (III) and compound (V) which is intermediate thereof]

### (Production Method D)

As an aspect, the compound (III) can be produced by a method shown in Production Scheme 6 below.

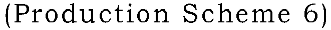

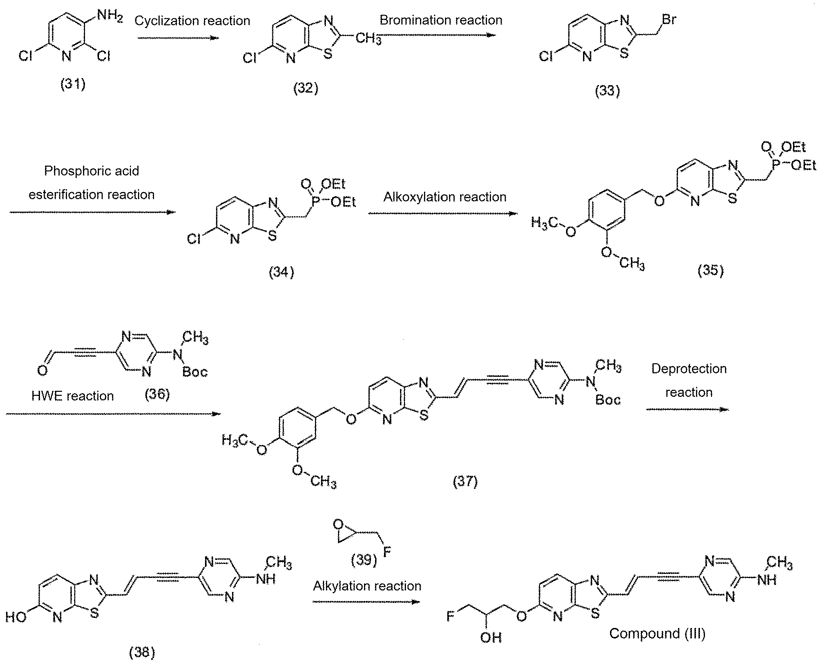

A compound (32) can be produced by a cyclization reaction with use of a compound (31).

A compound (33) can be produced by a bromination reaction of the compound (32).

A compound (34) can be produced by a reaction between the compound (33) and triethyl phosphite.

A compound (35) can be produced by an alkoxylation reaction of the compound (34) by 3,4-dimethoxybenzyl alcohol.

The compound (37) can be produced by a Horner-Wadsworth-Emmons reaction (HWE reaction) between the compound (35) and a compound (36) (described later).

A compound (38) can be produced by a deprotection reaction of the compound (37).

The compound (III) can be produced by an alkylation reaction of the compound (38) involving an oxirane ring-opening reaction of a compound (39).

The compound (36) used in Production Method D can be produced from a compound (40) by a method shown in Production Scheme 7 below.

In addition, when the compound (39) in which a fluorine atom is ¹⁸F, that is, [¹⁸F]2-(fluoromethyl)oxirane, is used in the alkylation reaction of the compound (38) involving an oxirane ring-opening reaction of the compound (39), the compound (III) in which a fluorine atom in a 3-fluoro-2-hydroxypropoxy group is a radioisotope can be produced.

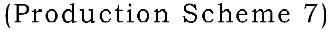

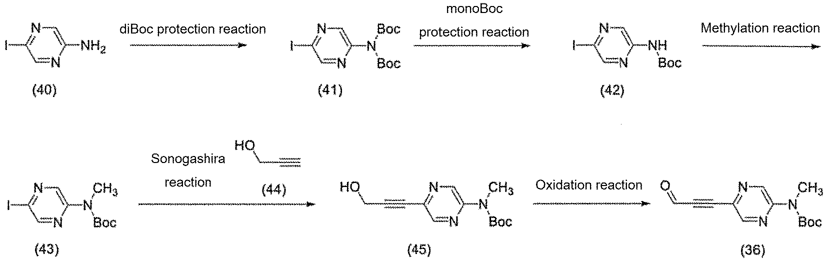

A compound (41) can be produced by an amino group protection (diBoc protection) reaction of the compound (40) by di-tert-butyl dicarbonate.

A compound (42) can be produced by deprotection reaction of one Boc group (monoBoc protection) in the compound (41) by a base such as potassium carbonate in a solvent such as methanol.

A compound (43) can be produced by a methylation reaction of the compound (42) by iodine methane or the like.

A compound (45) can be produced by a Sonogashira reaction between the compound (43) and propargyl alcohol (44).

The compound (36) can be produced by an oxidation reaction of the compound (45).

### (Production Method E)

As another aspect, the compound (V) in which R₄ is H (compound (V-i)) can be produced by a method shown in Production Scheme 8 below.

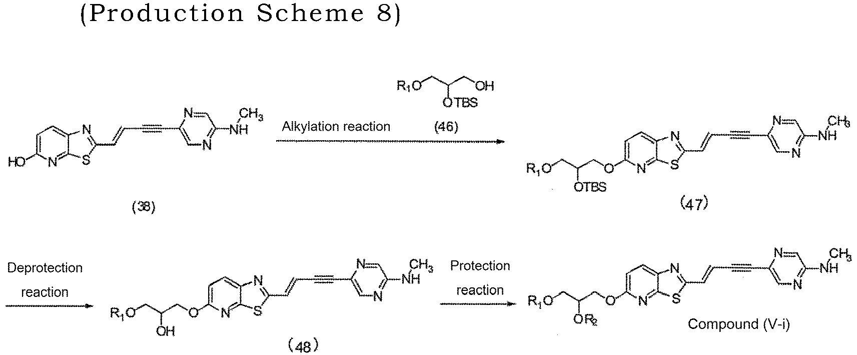

In Production Scheme 8, TBS represents a tertbutyldimethylsilyl group.

A compound (47) can be produced by an alkylation reaction, such as a Mitsunobu reaction, between the compound (38) and a compound (46) (described later).

A compound (48) can be produced by a deprotection reaction of the compound (47).

The compound (V-i) can be produced by a protection reaction of the compound (48).

A compound (46a) in which, in the compound (46) used in Production Method E, R₁ is a p-toluenesulfonyl group or a 4-nitrobenzenesulfonyl group can be produced from a compound (49) by a method shown in Production Scheme 9 below.

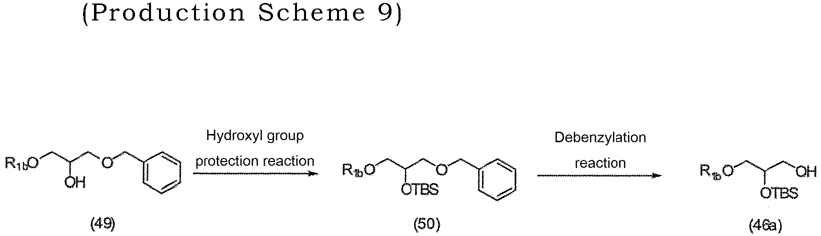

In Production Scheme 9, R_{1b} represents a p-toluenesulfonyl group or a 4-nitrobenzenesulfonyl group.

A compound (50) can be produced by a hydroxyl group protection reaction of the compound (49).

The compound (46a) can be produced by a debenzylation reaction of the compound (50).

### (Production Method F)

As a still another aspect, the compound (V-i) can be produced by a method shown in Production Scheme 10 below.

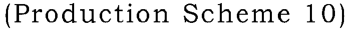

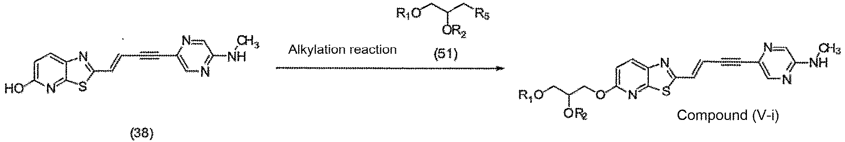

In Production Scheme 10, R₅ represents a halogen atom (e.g., a bromine atom or an iodine atom) or a leaving group such as a p-toluenesulfonyloxy group or a 4-nitrobenzenesulfonyloxy group.

A compound (V-i) can be produced by an alkylation reaction of the compound (38) by a compound (51) (described later).

A compound in which, in the compound (51) used in Production Method F, R₅ is a halogen atom, and R₁ is a p-toluenesulfonyl group or a 4-nitrobenzenesulfonyl group is a compound (21a) in Production Scheme 5 in [2. Method for producing compound (I) and compound (II) and compound (IV) which is intermediate of compound (I) and compound (II)] described above and can be produced by a method shown in Production Scheme 5.

### (Production Method F')

The compound (III) can be produced from the production intermediate compound (V) by a method shown in Production Scheme 10' below.

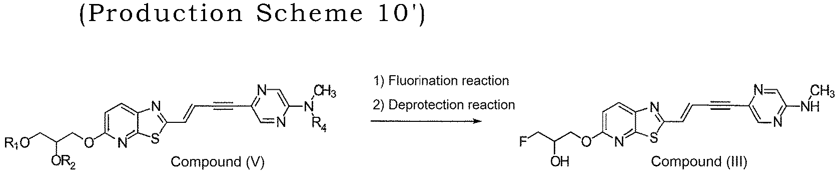

The compound (III) can be produced by a deprotection reaction after a fluorination reaction of the production intermediate compound (V) by potassium fluoride or the like. Further, the compound (III) which is a radioisotope can be produced with use of [¹⁸F]potassium fluoride or the like.

### [3. Method for producing compound (VI) which is intermediate of compound (I) and compound (II)]

### (Production Method G)

As an aspect, the compound (VI) in which R₁ is a p-toluenesulfonyl group (tosyl group), and R₄ is a hydrogen atom (compound (VI-i)) can be produced by a method shown in production scheme 11 below.

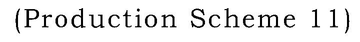

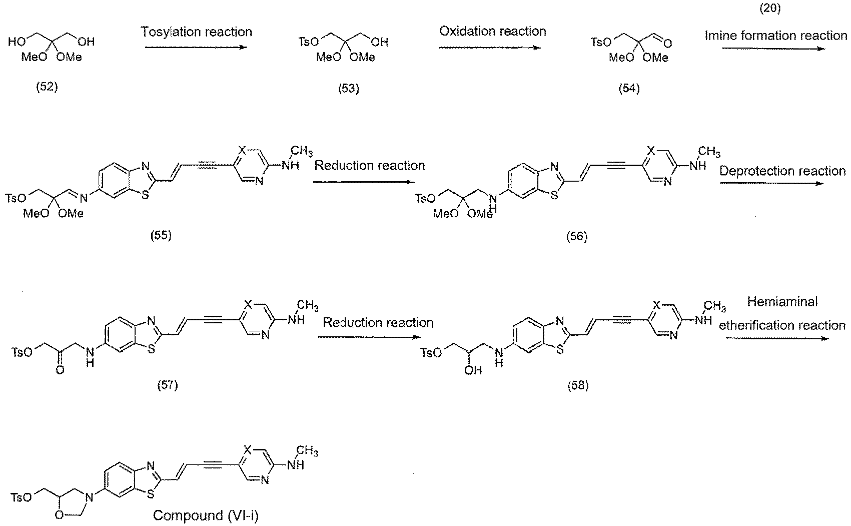

A compound (53) can be produced by a tosylation reaction of the compound (52).

A compound (54) can be produced by a hydroxyl group oxidation reaction of the compound (53).

A compound (55) can be produced by an imine formation reaction between the compound (20) and the compound (54).

A compound (56) can be produced by an imino group reduction reaction of the compound (55).

A compound (57) can be produced by a deprotection reaction of the compound (56).

A compound (58) can be produced by a carbonyl group reduction reaction of the compound (57).

The compound (VI-i) can be produced by a hemiaminal etherification reaction between an amino group and a hydroxyl group in the compound (58).

### (Production Method G')

The compounds (I) and (II) can be each produced from the production intermediate compound (VI) by a method shown in Production Scheme 11' below.

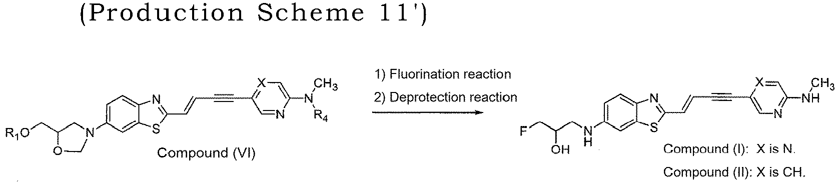

The compounds (I) and (II) can be each produced by a deprotection reaction after a fluorination reaction of the production intermediate compound (VI) by potassium fluoride or the like. Further, the compound (I) and the compound (II) which are radioisotopes can be produced with use of [¹⁸F]potassium fluoride or the like.

### [a-synuclein aggregate binding agent]

In an embodiment, the present invention provides an α-synuclein aggregate binding agent. The α-synuclein aggregate binding agent (hereinafter also referred to as "binding agent") in accordance with the present embodiment contains a compound (I), a compound (II), or a compound (III), a pharmaceutically acceptable salt thereof, or a solvate thereof.

The compound (I), the compound (II), and the compound (III) each have higher binding selectivity with respect to α-synuclein aggregates than with respect to aggregates of a tau protein and aggregates of an amyloid-β peptide. Similarly, the pharmaceutically acceptable salt of each of the compound (I), the compound (II), and the compound (III) and the solvate of each of the compound (I), the compound (II), and the compound (III) have higher binding selectivity with respect to α-synuclein aggregates than with respect to aggregates of a tau protein and aggregates of an amyloid-β peptide. The compound (I), the compound (II), and the compound (III) each fluoresce. Further, as described above, in each of the compound (I), the compound (II), and the compound (III), at least one atom can be a radioisotope thereof. Therefore, the binding agent in accordance with the present embodiment can be used as a molecular probe for optical imaging or radiological imaging of α-synuclein aggregates accumulated in the brain.

The α-synuclein aggregate binding agent can contain a pharmaceutically acceptable carrier. Examples of the pharmaceutically acceptable carrier include water, saline, physiological saline, phosphate buffered saline (PBS), sodium chloride injection solutions, Ringer's injection solutions, isotonic dextrose injection solutions, sterile water injection solutions, dextrose, and lactated Ringer's injection solutions.

The amounts of the compound (I), the compound (II), or the compound (III), a pharmaceutically acceptable salt thereof, or a solvate thereof and the pharmaceutically acceptable carrier contained in the α-synuclein aggregate binding agent are not particularly limited. These amounts are determined based on various factors such as: the compound to be used: the age, weight, health conditions, sex, and diet of a mammal to which administration is carried out; the number of times of administration and the route of the administration; the period of treatment; and other drugs that are concurrently used. The amount of the pharmaceutically acceptable carrier can be 1% by weight to 99% by weight of the α-synuclein aggregate binding agent. The α-synuclein aggregate binding agent is prepared such that, for example, the compound (I), the compound (II), or the compound (III) can be administered in an amount of 5 ng/kg to 5 mg/kg per weight (kg) of a subject. Preferably, the lower limit of the amount of the compound (I), the compound (II), or the compound (III) is not less than 5 ng/kg, not less than 0.01 mg/kg, not less than 0.05 mg/kg, or not less than 0.1 mg/kg. The upper limit of the amount of the compound (I) or the compound (II) is not more than 5 mg/kg, not more than 3 mg/kg, not more than 1 mg/kg, or not more than 20 µg/kg.

### [Composition for optical imaging of α-synuclein aggregates]

In an embodiment, the present invention provides a composition for optical imaging of α-synuclein aggregates. The composition for optical imaging of α-synuclein aggregates in accordance with the present embodiment (hereinafter also referred to simply as "composition for optical imaging") contains the above-described binding agent in accordance with the present embodiment. The optical imaging encompasses in vitro, ex vivo, and in vivo imaging.

Examples of the optical imaging include fluorescence microscope measurement, multiphoton imaging, two-photon imaging, and near infrared fluorescence imaging.

The composition for optical imaging can contain a pharmaceutically acceptable carrier. Examples of the pharmaceutically acceptable carrier include water, saline, physiological saline, phosphate buffered saline (PBS), sodium chloride injection solutions, Ringer's injection solutions, isotonic dextrose injection solutions, sterile water injection solutions, dextrose, and lactated Ringer's injection solutions.

The amounts of the compound (I), the compound (II), or the compound (III), a pharmaceutically acceptable salt thereof, or a solvate thereof and the pharmaceutically acceptable carrier contained in the composition for optical imaging are not particularly limited. These amounts are determined based on various factors such as: the compound to be used: the age, weight, health conditions, sex, and diet of a mammal to which administration is carried out; the number of times of administration and the route of the administration; the period of treatment; and other drugs that are concurrently used. The amount of the pharmaceutically acceptable carrier can be 1% by weight to 99% by weight of the composition for optical imaging. The composition for optical imaging is prepared such that, for example, the compound (I), the compound (II), or the compound (III) can be administered in an amount (mg) of 0.01 mg/kg to 5 mg/kg, preferably 0.05 mg/kg to 3 mg/kg, and still more preferably 0.1 mg/kg to 1 mg/kg, per weight (kg) of a subject.

[Composition for radiological imaging of α-synuclein aggregates]

In an embodiment, the present invention provides a composition for radiological imaging of α-synuclein aggregates. The composition for radiological imaging of α-synuclein aggregates in accordance with the present embodiment (hereinafter also referred to simply as "composition for radiological imaging") contains the above-described binding agent in accordance with the present embodiment that contains one selected from the compound (I), the compound (II), and the compound (III) each being a compound in which at least one atom is a radioisotope thereof. The radiological imaging encompasses in vitro, ex vivo, and in vivo imaging.

Examples of the radiological imaging include positron emission tomography (PET), single-photon emission computed tomography (SPECT), and autoradiography.

The composition for radiological imaging can contain a pharmaceutically acceptable carrier. Examples of the pharmaceutically acceptable carrier include water, saline, physiological saline, phosphate buffered saline (PBS), sodium chloride injection solutions, Ringer's injection solutions, isotonic dextrose injection solutions, sterile water injection solutions, dextrose, and lactated Ringer's injection solutions.

The amounts of the compound (I), the compound (II), or the compound (III), a pharmaceutically acceptable salt thereof, or a solvate thereof and the pharmaceutically acceptable carrier contained in the composition for radiological imaging are not particularly limited. These amounts are determined based on various factors such as: the compound to be used: the age, weight, health conditions, sex, and diet of a mammal to which administration is carried out; the number of times of administration and the route of the administration; the period of treatment; and other drugs that are concurrently used. The amount of the pharmaceutically acceptable carrier can be 1% by weight to 99% by weight of the composition for radiological imaging. The composition for radiological imaging is prepared such that, for example, the compound (I), the compound (II), or the compound (III) can be administered in an amount of 5 ng/kg to 5 mg/kg and preferably 5 ng/kg to 20 µg/kg per weight (kg) of a subject.

### [Diagnostic agent for diseases associated with α-synuclein aggregates or companion diagnostic agent for treating or preventing those diseases]

In an embodiment, the present invention provides a diagnostic agent for diseases associated with α-synuclein aggregates or a companion diagnostic agent for treating or preventing the diseases. The diagnostic agent for diseases associated with α-synuclein aggregates or the companion diagnostic agent for treating or preventing the diseases (hereinafter also referred to "companion diagnostic agent") in accordance with the present embodiment contains the above-described binding agent in accordance with the present embodiment. The companion diagnostic agent for treatment is a diagnostic agent for, when a disease is found, determining whether treatment would be possible or not. The companion diagnostic agent for prevention is a diagnostic agent for, when a precursor state of a diseases is found, predicting the future onset or determining whether prevention for suppressing the onset would be possible or not.

By collating data on the amount and/or distribution of α-synuclein aggregates in the brain of a subject, which is obtained by using the diagnostic agent of the present embodiment, with previously-obtained correlation between a disease and the amount and/or distribution of α-synuclein aggregates, it is possible to diagnose the condition of the disease in the subject (specifically, the presence or absence of the disease, the severity of the disease, the possibility of having episode, and the like).

Moreover, by collating data on the amount and/or distribution of α-synuclein aggregates in the brain of a subject, which is obtained by using the companion diagnostic agent of the present embodiment, with previously-obtained correlation between a disease and the amount and/or distribution of α-synuclein aggregates in brain, it is possible to assess the disease condition of the subject. Therefore, it is possible to plan for prevention/treatment of the disease (such as the type, combination, dosage, usage, and the like of a preventive/therapeutic agent to be administered) on the basis of the disease condition.

An embodiment of the present invention also relates to a medicament for treating or preventing a disease associated with α-synuclein aggregates, the medicament being administered in a dosage regimen based on data pertaining to the amount and/or distribution of α-synuclein aggregates in brain which is obtained by companion diagnosis.

### [Diagnostic kit for diseases associated with substance accumulated in brain]

A diagnostic kit, in accordance with an embodiment of the present invention, for a disease associated with a substance accumulated in brain (hereinafter also referred to as "diagnostic kit") contains the above-described binding agent in accordance with the present embodiment.

The substance accumulated in the brain includes at least α-synuclein aggregates. The substance further includes aggregates of tau proteins and aggregates of amyloid-β peptides.

The disease associated with a substance accumulated in the brain includes at least diseases associated with α-synuclein aggregates. The diseases include Parkinson's disease, dementia with Lewy bodies (DLB), and multiple system atrophy (MSA). The disease associated with a substance accumulated in the brain also include Alzheimer's disease (AD) and frontotemporal lobar degeneration, each of which is a disease associated with aggregates of a tau protein or an amyloid-β peptide.

The binding agent in accordance with the present embodiment has higher binding selectivity with respect to α-synuclein aggregates than with respect to aggregates of a tau protein and aggregates of an amyloid-β peptide. In contrast, the inventors of the present invention found that the compound disclosed in Patent Literature 1, for example, has higher binding performance with respect to aggregates of a tau protein than with respect to α-synuclein aggregates.

In an aspect of the present embodiment, the diagnostic kit can contain both (i) the compound (I), the compound (II), or the compound (III), a pharmaceutically acceptable salt thereof, or a solvate thereof, each of which has high binding selectivity with respect to α-synuclein aggregates, and (ii) another compound which has high binding selectivity with respect to tau protein aggregates (e.g. the compound disclosed in Patent Literature 1). Such a diagnostic kit makes it possible to, by comparing a result (the amount and/or distribution of detected light or radioactivity) of detection of the former with a result of detection of the latter, determine which substance is present in each imaged area. Specifically, it is possible to classify α-synuclein aggregates and tau protein aggregates, and further possible to quantify the abundance of α-synuclein aggregates and tau protein aggregates. Thus, it is possible to diagnose, with high accuracy, a disease associated with α-synuclein aggregates and/or a disease associated with tau protein aggregates. For example, a ratio between binding performance with respect to α-synuclein aggregates and binding performance with respect to tau protein aggregates is previously determined for each of the binding agent in accordance with the present embodiment and a compound which has high binding selectivity with respect to tau protein aggregates (e.g., the compound disclosed in Patent Literature 1). By then measuring, in each area in the brain of a subject, a ratio between the amount of light or radioactivity after administration of the former and the amount of light or radioactivity after administration of the latter, it is possible to distinguish α-synuclein aggregates from tau protein aggregates in each area in the brain and quantify the abundance of those substances, based on a relation between the previously determined ratio and the measured ratio.

The diagnostic kit in accordance with the present embodiment can be further combined with an imaging agent for an amyloid-β peptide to thereby obtain a diagnostic kit. Such a diagnostic kit which is obtained by combining the diagnostic kit in accordance with the present embodiment further with an imaging agent for an amyloid-β peptide makes it possible to determine substances present in each imaged area. Specifically, it is possible to distinguish between α-synuclein aggregates, tau protein aggregates, and amyloid-β aggregates, and further possible to quantify the abundance of the α-synuclein aggregates, the tau protein aggregates, and the amyloid-β aggregates. Thus, it is possible to diagnose, with high accuracy, a disease associated with α-synuclein aggregates, a disease associated with tau protein, and/or a disease associated with amyloid-β peptides.

An embodiment of the present invention also relates to a medicament for treating or preventing a disease associated with α-synuclein aggregates, the medicament being administered in a dosage regimen based on data pertaining to the amount and/or distribution of a substance, including α-synuclein aggregates and accumulated in the brain, which is obtained by the diagnostic kit in accordance with the present embodiment.

### [Optical imaging method]

In an embodiment, the present invention provides a method for carrying out optical imaging. The method for carrying out optical imaging in accordance with the present embodiment includes a step of detecting light which has a second wavelength and which is emitted from the brain of a living subject, after externally irradiating the brain with light which has a first wavelength, wherein the above-described binding agent in accordance with the present embodiment has been administered to the living subject, and the first wavelength and the second wavelength are different from each other.

In a case where an effective amount of the binding agent is administered to a subject, the binding agent that has been delivered to the brain of the subject binds to α-synuclein aggregates present in the brain. By externally irradiating the brain of the subject to which the binding agent has been administered with light which has the first wavelength and excites the binding agent, followed by detecting light (e.g., fluorescence) which is emitted from the binding agent in the brain and has the second wavelength, it is possible to carry out optical imaging of the α-synuclein aggregates.

The subject include mammals. Examples of the mammals include humans, rats, mice, rabbits, guinea pigs, hamsters, monkeys, dogs, ferrets, and minipigs. Preferably, the mammals are humans.

An administration method is not particularly limited, and examples of the administration method include oral administration and parenteral administration such as intravenous administration and intraperitoneal administration. Preferably, intravenous or intraperitoneal administration is employed. Most preferably, intravenous administration is employed. As a dose, the amount (mg) of the compound (I), the compound (II), or the compound (III) per kilogram of body weight of a subject is preferably 0.01 mg/kg to 5 mg/kg, 0.05 mg/kg to 3 mg/kg, or 0.1 mg/kg to 1 mg/kg, and most preferably 0.1 mg/kg to 1 mg/kg.

### [Radiological imaging method]

In an embodiment, the present invention provides a method for carrying out radiological imaging. The method for carrying out radiological imaging in accordance with the present embodiment includes a step of detecting radioactivity emitted from the brain of a living subject to which the binding agent in accordance with the present embodiment, which contains the compound (I), the compound (II), or the compound (III), in each of which at least one atom is a radioisotope thereof, a pharmaceutically acceptable salt thereof, or a solvate thereof, has been administered.

In a case where an effective amount of the binding agent is administered to a living subject, the binding agent that has been delivered to the brain of the living subject binds to α-synuclein aggregates present in the brain. By detecting radioactivity emitted from the binding agent in the brain, it is possible to carry out radiological imaging of the α-synuclein aggregates.

The subject include mammals. Examples of the mammals include humans, rats, mice, rabbits, guinea pigs, hamsters, monkeys, dogs, ferrets, and minipigs. Preferably, the mammals are humans.

An administration method is not particularly limited, and examples of the administration method include oral administration and parenteral administration such as intravenous administration and intraperitoneal administration. Preferably, intravenous or intraperitoneal administration is employed. Most preferably, intravenous administration is employed. As a dose, the amount (mg) of the compound (I), the compound (II), or the compound (III) per kilogram of body weight of a subject is preferably 5 ng/kg to 5 mg/kg, and more preferably 5 ng to 20 µg/kg. An applied radiation dose is preferably 37 MBq to 7.4 GBq, and more preferably 370 MBq to 3700 MBq, per individual.

### [Method for screening therapeutic or preventive agent for disease associated with α-synuclein aggregates in brain]

In an embodiment, the present invention provides a method for screening a therapeutic or preventive agent for a disease associated with α-synuclein aggregates in brain. The method for screening a therapeutic or preventive agent for a disease associated with α-synuclein aggregates in brain in accordance with the present embodiment (hereinafter also referred to as "screening method") includes a step of selecting a candidate agent on the basis of a difference, in amount and/or distribution of light or radioactivity which is detected by the optical imaging method or the radiological imaging method in accordance with the present embodiment, between before and after administration of the candidate agent to a subject.

The disease associated with α-synuclein aggregates in brain is similar to at least diseases associated with α-synuclein aggregates described in the foregoing [Diagnostic kit for disease associated with substance accumulated in brain].

The subject and an administration method are similar to those described in the foregoing [Optical imaging method] and [Radiological imaging method].

For example, in a case where, after administration of a candidate agent, the amount (intensity) of light (such as fluorescence) or radioactivity from the binding agent is reduced as compared with that before the administration of the candidate agent, the candidate agent can be useful as a therapeutic agent for the disease or symptom.

Moreover, in comparison of the amount and/or distribution of light or radioactivity detected in a subject with those/that in another healthy mammal, when the amount and/or distribution in the subject after administration of a candidate agent becomes closer to those/that in the healthy mammal, the candidate agent can be useful as a therapeutic agent for the disease or a symptom.

For example, data pertaining to the amount (intensity) and/or distribution of light (such as fluorescence) or radioactivity from the binding agent in a subject before and after the administration of a candidate agent is compared with an increase in the amount and/or changes in distribution of the same before and after the onset of a disease associated with α-synuclein aggregates in brain which has been preliminarily observed in a mammal without administration of the candidate agent. In a case where an increase in the amount of the light or radioactivity which is observed after the onset of the disease is suppressed by administration of the candidate agent and/or in a case where, after administration of the candidate agent, the amount of the light or radioactivity is similar to that before administration and is close to that in a healthy mammal, the candidate agent can be useful as a preventive compound for the disease or symptom. Similarly, in a case where changes in distribution of the light or radioactivity observed after the onset of a disease is suppressed by administration of the candidate agent and/or in a case where, after administration of the candidate agent, the changes in distribution of the light or radioactivity are similar to those before administration and are close to those in a healthy mammal, the candidate agent can be useful as a preventive agent for the disease or a symptom.

### [Method for quantifying or determining accumulation of α-synuclein aggregates in brain]

In an embodiment, the present invention provides a method for quantifying or determining accumulation of α-synuclein aggregates in brain. The method for quantifying or determining accumulation of α-synuclein aggregates in brain in accordance with the present embodiment includes a step of detecting light which has a second wavelength and which is emitted from the brain of a living subject, after externally irradiating the brain with light which has a first wavelength, wherein the above-described binding agent which contains the compound (I), the compound (II), or the compound (III), a pharmaceutically acceptable salt thereof, or a solvate thereof has been administered to the living subject, and the first wavelength and the second wavelength are different from each other. Based on the amount and/or distribution of the detected light, accumulation of α-synuclein aggregates in the brain is quantified or determined. This method is a method for quantifying or determining accumulation of α-synuclein aggregates in brain by optical imaging.

The subject and an administration method are similar to those described in the foregoing [Optical imaging method].

By determining a difference(s) between the amount and/or distribution of light detected in a subject and those/that in a healthy mammal, it is possible to quantify accumulation of α-synuclein aggregates in the brain and to determine the presence or absence of the accumulation of α-synuclein aggregates in the brain.

In another aspect of the method for quantifying or determining accumulation of α-synuclein aggregates in brain in accordance with the present embodiment, the method includes a step of detecting radioactivity emitted from the brain of a living subject to which the binding agent in accordance with the present embodiment, which contains the compound (I), the compound (II), or the compound (III), in each of which at least one atom is a radioisotope thereof, a pharmaceutically acceptable salt thereof, or a solvate thereof, has been administered. On the basis of the amount and/or distribution of the detected radioactivity, accumulation of α-synuclein aggregates in the brain is quantified or determined. This method is a method for quantifying or determining accumulation of α-synuclein aggregates in brain by radiological imaging.

The subject and an administration method are similar to those described in the foregoing [Radiological imaging method].

By determining a difference(s) between the amount and/or distribution of radioactivity detected in a subject and those/that in a healthy mammal, it is possible to quantify accumulation of α-synuclein aggregates in the brain and to determine the presence or absence of the accumulation of α-synuclein aggregates in the brain.

### [Method for classifying substance(s) accumulated in brain and determining accumulation of substance(s)]

In an embodiment, the present invention provides a method for classifying a substance(s) accumulated in brain and determining accumulation of the substance(s). The method for classifying a substance(s) accumulated in brain and determining accumulation of the substance(s) in accordance with an embodiment of the present invention includes: a first step of detecting light which has a second wavelength and which is emitted from the brain of a living subject, after externally irradiating the brain with light which has a first wavelength, wherein the above-described binding agent in accordance with the present embodiment, which contains the compound (I), the compound (II), or the compound (III), a pharmaceutically acceptable salt thereof, or a solvate thereof, has been administered to the living subject, and the first wavelength and the second wavelength are different from each other; and a second step of detecting light which has a fourth wavelength and which is emitted from the brain of the living subject, after externally irradiating the brain with light which has a third wavelength, wherein a compound that has high binding selectivity with respect to tau protein aggregates (e.g., the compound disclosed in Patent Literature 1) has been administered to the living subject at a time point differing from the administration in the first step, and the third wavelength and the fourth wavelength are different from each other. Based on the data pertaining to the amount and/or distribution of the light detected in each of the first step and the second step, a substance(s) accumulated in the brain is/are classified, and accumulation of the substance(s) is determined. This method is a method for, by optical imaging, classifying a substance(s) accumulated in brain and determining accumulation of the substance(s).

The subject and an administration method are similar to those described in the foregoing [Optical imaging method].

The method includes both the first step of detecting light which is emitted from the brain and which results from administration of the binding agent having high binding selectivity with respect to α-synuclein aggregates and the second step of detecting light which is emitted from the brain of the same subject and which results from administration of the substance having high binding selectivity with respect to tau protein aggregates. Therefore, by comparing a result (the amount and/or distribution of the detected light) of detection in the first step with a result of detection in the second step, it is possible to classify a substance(s) accumulated in the brain into either α-synuclein aggregates or tau protein aggregates, and to determine the accumulation of α-synuclein aggregates and tau protein aggregates.

In another aspect of the method for classifying a substance(s) accumulated in the brain and determining accumulation of the substance(s) in accordance with the present embodiment includes: a first step of detecting radioactivity emitted from the brain of a living subject to which the binding agent in accordance with the present embodiment, which contains the compound (I), the compound (II), or the compound (III), in each of which at least one atom is a radioisotope thereof, a pharmaceutically acceptable salt thereof, or a solvate thereof, has been administered; and a second step of detecting radioactivity which is emitted from the brain of the subject to which a radioactive compound that has high binding selectivity with respect to tau protein aggregates (e.g., the compound disclosed in Patent Literature 1) has been administered at a time point differing from that in the first step. Based on the data pertaining to the amount and/or distribution of the radioactivity detected in the first step and data pertaining to the amount and/or distribution of the radioactivity detected in the second step, a substance(s) accumulated in the brain is/are classified, and accumulation of the substance(s) is determined. This method is a method for, by radiological imaging, classifying a substance(s) accumulated in the brain and determining accumulation of the substance(s).

The subject and an administration method are similar to those described in the foregoing [Radiological imaging method].

The method includes both the first step of detecting radioactivity which is emitted from the brain and which results from administration of the binding agent having high binding selectivity with respect to α-synuclein aggregates and the second step of detecting radioactivity which is emitted from the brain of the same subject and which results from administration of the substance having high binding selectivity with respect to tau protein aggregates. Therefore, by comparing a result (the amount and/or distribution of the detected radioactivity) of detection in the first step with a result of detection in the second step, it is possible to carry out classification as to whether a substance(s) accumulated in the brain is/are α-synuclein aggregates and/or tau protein aggregates, and possible to determine accumulation of α-synuclein aggregates and tau protein aggregates.

### (Summary)

The embodiments of the present invention are summarized below.

An aspect of the present invention is a compound represented by the following formula (I), (II), or (III), a pharmaceutically acceptable salt thereof, or a solvate thereof.

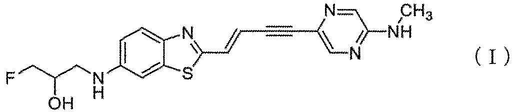

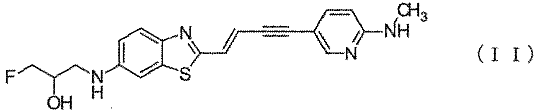

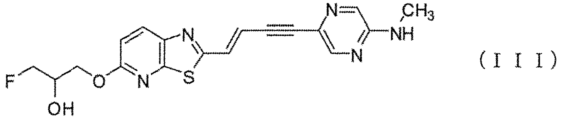

An aspect of the present invention is the compound, the pharmaceutically acceptable salt thereof, or the solvate thereof, wherein at least one atom in the compound represented by the formula (I), (II), or (III) is a radioisotope thereof.

An aspect of the present invention is an α-synuclein aggregate binding agent containing the compound, the pharmaceutically acceptable salt, or the solvate thereof.

An aspect of the present invention is a composition for optical imaging of α-synuclein aggregates, the composition containing the α-synuclein aggregate binding agent.

An aspect of the present invention is a composition for radiological imaging of α-synuclein aggregates, the composition containing the α-synuclein aggregate binding agent.

An aspect of the present invention is a method for carrying out optical imaging of α-synuclein aggregates in brain, the method including a step of detecting light which has a second wavelength and which is emitted from the brain of a living subject, after externally irradiating the brain with light which has a first wavelength, wherein the α-synuclein aggregate binding agent has been administered to the living subject, and the first wavelength and the second wavelength are different from each other.

An aspect of the present invention is a method for carrying out radiological imaging of α-synuclein aggregates in brain, the method including the step of detecting radioactivity which is emitted from the brain of a living subject to which the α-synuclein aggregate binding agent has been administered.

An aspect of the present invention is an intermediate for synthesizing the compound, the intermediate being represented by the following formula (IV) or (V): 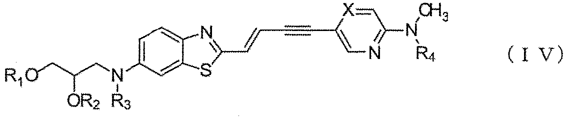 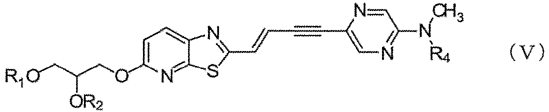 wherein, in the formulae (IV) and (V),
R₁ is a 4-nitrobenzenesulfonyl group, a p-toluenesulfonyl group, or a methanesulfonyl group,
R₂ is a tetrahydro-2H-pyran-2-yl group or a methoxymethyl group, and
R₄ is a hydrogen atom, a tert-butoxycarbonyl group, or a 2,4-dimethoxybenzyl group,
wherein, in the formula (IV),
X is a nitrogen atom (N) or an unsubstituted carbon atom (CH), and
R₃ is a hydrogen atom, a tert-butoxycarbonyl group, or a 2,4-dimethoxybenzyl group.

An aspect of the present invention is an intermediate for synthesizing the compound, the intermediate being represented by the following formula (VI): 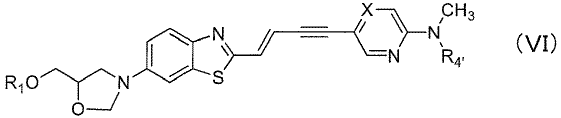 wherein:
R₁ is a 4-nitrobenzenesulfonyl group, a p-toluenesulfonyl group, or a methanesulfonyl group;
R_{4'} is a hydrogen atom or a tert-butoxycarbonyl group; and
X is a nitrogen atom (N) or an unsubstituted carbon atom (CH).

The present invention is not limited to the embodiments, but can be altered by a skilled person in the art within the scope of the claims. The present invention also encompasses, in its technical scope, any embodiment derived by combining technical means disclosed in differing embodiments.

### Examples

### (Production Examples)

In production examples (Reference Examples and Examples) below, the "room temperature" generally indicates a temperature of approximately 10°C to approximately 35°C. The "%" indicates "% by weight", unless otherwise specified. In description of a compound used in each production example, the expression "produced in Reference Example (Example) X" is intended to also include a case of "produced in a manner similar to that in Reference Example (Example) X".

Elution in column chromatography in each production example was carried out under observation by thin layer chromatography (TLC), unless otherwise specified. In the TLC observation, 60 F254, manufactured by Merck, was used as a TLC plate, and a solvent used as an elution solvent in the column chromatography was used as a developing solvent. For detection, a UV detector was employed.

ACD/SpecManager (product name) software and the like were used to analyze ¹H NMR, and values obtained by the analysis were shown. A proton peak which was extremely gentle, such as those of a hydroxyl group and an amino group, may not be shown.

In Examples below, the following abbreviations are used.
Boc: tert-butoxycarbonyl group
DMF: N,N-dimethylformamide
DMSO: dimethyl sulfoxide
DMSO-d₆: deuterated dimethyl sulfoxide
¹H NMR: proton nuclear magnetic resonance
M: molar concentration
MeOH: methanol
TBS: tert-butyldimethylsilyl group
TEA: triethylamine
TFA: trifluoroacetic acid
THF: tetrahydrofuran
THP: tetrahydropyranyl group
TMS: trimethylsilyl group
Ts: p-toluenesulfonyl group

### Reference Example T001: production of di-tert-butyl (5-iodopyrazin-2-yl)-2-imidodicarbonate

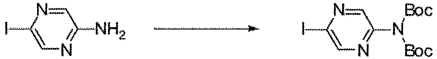

To a 0°C solution of 5-iodopyrazin-2-amine (CAS [886860-50-0]) (9.80 g) in THF (50 ml) were added a solution of di-tert-butyl dicarbonate (22.3 g) in THF (25 ml) and 4-dimethylaminopyridine (1.35 g). The mixture was stirred at 0°C to room temperature overnight, and diluted with water and ethyl acetate. The insoluble was removed by filtration, and the filtrate was extracted with ethyl acetate. The extract was washed with water and brine, dried over anhydrous sodium sulfate, passed through a short silica gel column, and concentrated under reduced pressure to obtain the title compound (17.74 g) as a brown solid.
¹H NMR (300 MHz, DMSO-d₆) δ 1.40 (18H, s), 8.65 (1H, d, J=1.3 Hz), 8.90 (1H, d, J=1.4 Hz).

### Reference Example T002: production of tert-butyl (5-iodopyrazin-2-yl)carbamate

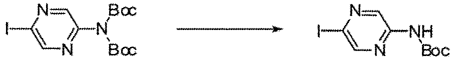

To a solution of di-tert-butyl (5-iodopyrazin-2-yl)-2-imidodicarbonate (17.7 g) produced in Reference Example T001 in MeOH (200 ml), was added potassium carbonate (6.97 g) at room temperature. The mixture was stirred at room temperature overnight, concentrated to approximately a quarter volume under reduced pressure, cooled to 0°C, neutralized with 5% citric acid solution, and diluted with water. The precipitated solid was collected by filtration, washed with water, and dried to obtain the title compound (11.4 g) as a brown solid.
¹H NMR (300 MHz, DMSO-d₆) δ 1.48 (9H, s), 8.58 (1H, d, J=1.5 Hz), 8.89 (1H, d, J=1.5 Hz), 10.33 (1H, brs).

### Reference Example T003: production of tert-butyl (5-iodopyrazin-2-yl)(methyl)carbamate

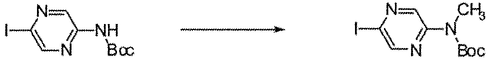

To a 0°C solution of tert-butyl (5-iodopyrazin-2-yl)carbamate (2.00 g) produced in Reference Example T002 and cesium carbonate (3.04 g) in DMF (15 ml), was added methyl iodide (0.504 ml). The mixture was stirred at 0°C to room temperature overnight, cooled to 0°C, and diluted with water. The precipitated solid was collected by filtration, washed with water, and dried to obtain the title compound (1.95 g) as a beige solid.
¹H NMR (300 MHz, DMSO-d₆) δ 1.49 (9H, s), 3.27 (3H, s), 8.71 (1H, d, J=1.5 Hz), 8.83 (1H, d, J=1.5 Hz).

### Reference Example T004: production of tert-butyl methyl(5-((trimethylsilyl)ethynyl)pyrazin-2-yl)carbamate

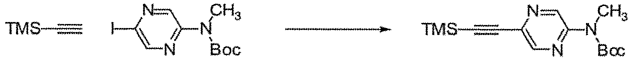

To a solution of tert-butyl (5-iodopyrazin-2-yl)(methyl)carbamate (1.95 g) produced in Reference Example T003, ethynyltrimethylsilane (1.62 ml), and dichlorobis(triphenylphosphine)palladium (II) (123 mg) in THF (6 ml) and TEA (6 ml), was added copper iodide (I) (66 mg) at room temperature under nitrogen atmosphere. The mixture was stirred at room temperature overnight, cooled to 0°C, diluted with ethyl acetate, neutralized with 5% citric acid solution, and subjected to Celite filtration. The filtrate was extracted with ethyl acetate. The extract was washed with water and brine, dried over anhydrous sodium sulfate, passed through a short silica gel column, and concentrated under reduced pressure. The residue was purified by silica gel column chromatography (hexane/ethyl acetate) to obtain the title compound (1.98 g, including 22% by weight of 1,4-bis(trimethylsilyl)but-1,3-diyn) as a yellow solid.
¹H NMR (300 MHz, DMSO-d₆) δ 0.26 (9H, s), 1.50 (9H, s), 3.31 (3H, s), 8.55 (1H, d, J=1.5 Hz), 8.99 (1H, d, J=1.5 Hz).

### Reference Example T005: production of tert-butyl (5-ethynylpyrazin-2-yl) (methyl) carbamate

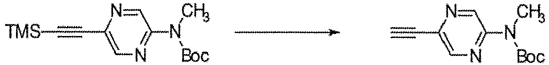

To a 0°C solution of tert-butyl methyl(5-((trimethylsilyl)ethynyl)pyrazin-2-yl)carbamate (1.98 g, including 22% by weight of 1,4-bis(trimethylsilyl)but-1,3-diyn) produced in Reference Example T004 in THF (20 ml), was added tetra-n-butylammonium fluoride (1M THF solution, 11.1 ml). The mixture was stirred at 0°C for 0.5 hours, and diluted with water and ethyl acetate, and extracted with ethyl acetate. The extract was washed with water and brine, dried over anhydrous sodium sulfate, and concentrated under reduced pressure. The residue was purified by silica gel column chromatography (hexane/ethyl acetate) to obtain the title compound (1.11 g) as a yellow solid.
¹H NMR (300 MHz, DMSO-d₆) δ 1.50 (9H, s), 3.31 (3H, s), 4.54 (1H, s), 8.58 (1H, d, J=1.5 Hz), 8.99 (1H, d, J=1.3 Hz).

### Reference Example T006: production of 1-(benzo[d]thiazol-6-ylamino)-3-fluoropropan-2-ol

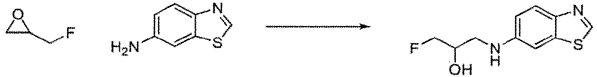

A solution of benzo[d]thiazol-6-amine (CAS [533-30-2]) (1.00 g) and 2-(fluoromethyl)oxirane (CAS [503-09-3]) (1.42 ml) in MeOH (10 ml) was stirred at 50°C overnight, and concentrated under reduced pressure. The residue was purified by silica gel column chromatography (hexane/ethyl acetate) to obtain the title compound (700 mg) as a colorless liquid.
¹H NMR (300 MHz, DMSO-d₆) δ 3.01-3.13 (1H, m), 3.14-3.26 (1H, m), 3.79-3.99 (1H, m), 4.27-4.60 (2H, m), 5.28 (1H, d, J=5.3 Hz), 5.98 (1H, t, J=6.0 Hz), 6.89 (1H, dd, J=8.7, 2.3 Hz), 7.16 (1H, d, J=2.3 Hz), 7.75 (1H, d, J=8.7 Hz), 8.90 (1H, s).

### Reference Example T007: production of N-(2-((tert-butyldimethylsilyl)oxy)-3-fluoropropyl)benzo[d]thiazol-6-amine

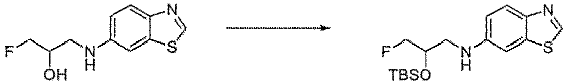

To a solution of 1-(benzo[d]thiazol-6-ylamino)-3-fluoropropan-2-ol (700 mg) produced in Reference Example T006 and 1H-imidazole (316 mg) in DMF (15 ml), was added tert-butyldimethylchlorosilane (606 mg) at room temperature. The mixture was stirred at room temperature overnight, cooled to 0°C, diluted with ethyl acetate and water, adjusted to pH approximately 5 with 5% citric acid solution, and extracted with ethyl acetate. The extract was washed with water and brine, dried over anhydrous sodium sulfate, and concentrated under reduced pressure. The residue was purified by silica gel column chromatography (hexane/ethyl acetate) to obtain the title compound (947 mg) as a colorless liquid.
¹H NMR (300 MHz, DMSO-d₆) δ 0.03 (3H, s), 0.03 (3H, s), 0.86 (9H, s), 3.04-3.30 (2H, m), 4.03-4.19 (1H, m), 4.27-4.62 (2H, m), 6.00 (1H, t, J = 6.2 Hz), 6.86 (1H, dd, J=8.8, 2.4 Hz), 7.15 (1H, d, J=2.3 Hz), 7.75 (1H, d, J=8.7 Hz), 8.90 (1H, s)

### Reference Example T008: production of tert-butyl benzo[d]thiazol-6-yl(2-((tert-butyldimethylsilyl)oxy)-3-fluoropropyl)carbamate

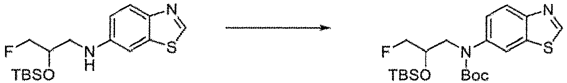

To a solution of N-(2-((tert-butyldimethylsilyl)oxy)-3-fluoropropyl)benzo[d]thiazol-6-amine (945 mg) produced in Reference Example T007 in THF (15 ml), were added di-tert-butyl dicarbonate (727 mg) and N,N-dimethylpyridin-4-amine (170 mg) at room temperature. The mixture was stirred at room temperature for 3 hours, followed by addition of di-tert-butyl dicarbonate (475 mg). The mixture was stirred at 60°C for 3 hours, cooled to 0°C, diluted with ethyl acetate and water, and extracted with ethyl acetate. The extract was washed with water and brine, dried over anhydrous sodium sulfate, and concentrated under reduced pressure. The residue was purified by silica gel column chromatography (hexane/ethyl acetate) to obtain the title compound (1.03 g) as a colorless liquid.
¹H NMR (300 MHz, DMSO-d₆) δ -0.08 (3H, s), -0.01 (3H, s), 0.77 (9H, s), 1.39 (9H, s), 3.66-3.88 (2H, m), 4.05-4.18 (1H, m), 4.20-4.56 (2H, m), 7.49 (1H, dd, J=8.7, 2.3 Hz), 8.03 (1H, d, J=9.0 Hz), 8.13 (1H, d, J=1.9 Hz), 9.36 (1H, s).

### Reference Example T009: production of tert-butyl (2-((tert-butyldimethylsilyl)oxy)-3-fluoropropyl)(2-formylbenzo[d]thiazol-6-yl)carbamate

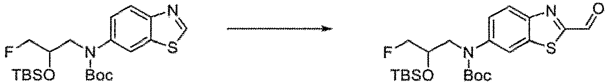

To a -78°C solution of tert-butyl benzo[d]thiazol-6-yl(2-((tert-butyldimethylsilyl)oxy)-3-fluoropropyl)carbamate (1.00 g) produced in Reference Example T008 in THF (18 ml), was added n-butyllithium (1.6M hexane solution, 1.70 ml). The mixture was stirred at -78°C for 0.5 hours, followed by addition of a solution of DMF (0.228 ml) in THF (2 ml). The mixture was stirred at -78°C for 1.5 hours, stirred at room temperature for 0.5 hours, cooled to 0°C, diluted with ethyl acetate, adjusted to pH approximately 5 with 5% citric acid solution, diluted with water, and extracted with ethyl acetate. The extract was washed with water and brine, dried over anhydrous sodium sulfate, and concentrated under reduced pressure. The residue was purified by silica gel column chromatography (hexane/ethyl acetate) to obtain the title compound (166 mg) as a colorless liquid.
¹H NMR (300 MHz, DMSO-d₆) δ -0.09 (3H, s), -0.01 (3H, s), 0.75 (9H, s), 1.41 (9H, s), 3.68-3.93 (2H, m), 4.07-4.22 (1H, m), 4.22-4.57 (2H, m), 7.67 (1H, dd, J=9.0, 2.3 Hz), 8.23 (1H, d, J=8.7 Hz), 8.26 (1H, d, J=1.9 Hz), 10.10 (1H, s).

### Reference Example T010: production of tert-butyl (E)-(2-(2-bromovinyl)benzo[d]thiazol-6-yl)(2-((tert-butyldimethylsilyl)oxy)-3-fluoropropyl)carbamate

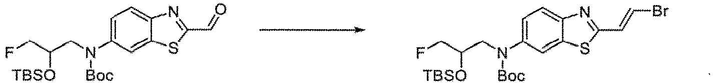

To a -78°C solution of (bromomethyl)triphenylphosphonium bromide (CAS [1034-49-7]) (228 mg) in THF (10 ml), was added pottasium tert-butoxide (1M THF solution, 0.522 ml). The mixture was stirred at -78°C for 2 hours, followed by addition of a solution of tert-butyl (2-((tert-butyldimethylsilyl)oxy)-3-fluoropropyl)(2-formylbenzo[d]thiazol-6-yl)carbamate (163 mg) produced in Reference Example T009 in THF (5 ml). The mixture was stirred at -78°C to room temperature for 2 hours, cooled to 0°C, diluted with ethyl acetate and water, neutralized with 5% citric acid solution, and extracted with ethyl acetate. The extract was washed with water and brine, dried over anhydrous sodium sulfate, and concentrated under reduced pressure. The residue was purified by silica gel column chromatography (hexane/ethyl acetate) to obtain the title compound (65 mg) as a colorless liquid.
¹H NMR (300 MHz, DMSO-d₆) δ -0.08 (3H, s), -0.02 (3H, s), 0.76 (9H, s), 1.39 (9H, s), 3.62-3.87 (2H, m), 4.05-4.19 (1H, m), 4.20-4.56 (2H, m), 7.47 (1H, dd, J=8.8, 2.1 Hz), 7.60 (1H, d, J=14.1 Hz), 7.83 (1H, d, J=13.9 Hz), 7.93 (1H, d, J=8.7 Hz), 8.07 (1H, d, J=2.3 Hz).

### Reference Example T011: production of tert-butyl (E)-(2-(4-(5-((tert-butoxycarbonyl)(methyl)amino)pyrazin-2-yl)but-1-en-3-yn-1-yl)benzo[d]thiazol-6-yl)(2-((tert-butyldimethylsilyl)oxy)-3-fluoropropyl)carbamate

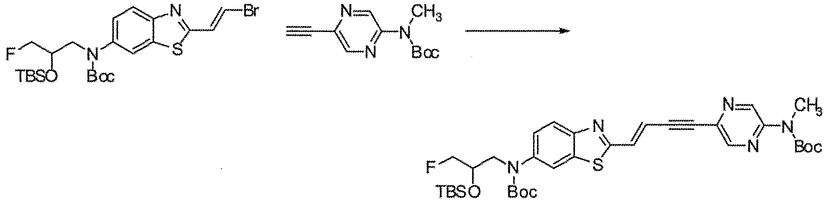

To a solution of tert-butyl (E)-(2-(2-bromovinyl)benzo[d]thiazol-6-yl)(2-((tert-butyldimethylsilyl)oxy)-3-fluoropropyl)carbamate (30 mg) produced in Reference Example T010, tert-butyl (5-ethynylpyrazin-2-yl)(methyl)carbamate (17 mg) produced in Reference Example T005, and dichlorobis(triphenylphosphine)palladium (II) (1.9 mg) in THF (1 ml) and TEA (2 ml), was added copper iodide (I) (1.0 mg) at room temperature under nitrogen atmosphere. The mixture was stirred at room temperature for 2 hours, diluted with ethyl acetate, adjusted to pH approximately 4 with 5% citric acid solution, and subjected to Celite filtration. The filtrate was extracted with ethyl acetate. The extract was washed with water and brine, dried over anhydrous sodium sulfate, passed through a short silica gel column, and concentrated under reduced pressure. The residue was purified by silica gel column chromatography (hexane/ethyl acetate) to obtain the title compound (22 mg) as a yellow solid.
¹H NMR (300 MHz, DMSO-d₆) δ -0.07 (3H, s), -0.01 (3H, s), 0.77 (9H, s), 1.40 (9H, s), 1.52 (9H, s), 3.34 (3H, s), 3.66-3.88 (2H, m), 4.06-4.57 (3H, m), 7.10 (1H, d, J=15.8 Hz), 7.50 (1H, brdd, J=8.7, 2.3 Hz), 7.53 (1H, d, J=16.2 Hz), 7.97 (1H, d, J=9.0 Hz), 8.10 (1H, d, J=2.3 Hz), 8.66 (1H, d, J=1.5 Hz), 9.07 (1H, d, J=1.5 Hz).

### Reference Example T012: production of tert-butyl (2-formylbenzo[d]thiazol-6-yl)carbamate

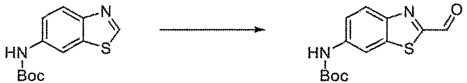

To a -78°C solution of tert-butyl benzo[d]thiazol-6-ylcarbamate (CAS [1192841-91-0]) (4.34 g) in THF (50 ml), was added n-butyllithium (1.6M hexane solution, 27.1 ml). The mixture was stirred at -78°C for 1 hour, followed by addition of a solution of DMF (5.37 ml) in THF (10 ml). The mixture was stirred at -78°C for 1.5 hours, stirred at room temperature for 0.5 hours, cooled to 0°C, diluted with ethyl acetate, adjusted to pH approximately 5 with 5% citric acid solution, diluted with water, and extracted with ethyl acetate. The extract was washed with water and brine, dried over anhydrous sodium sulfate, and concentrated under reduced pressure. The residual solid was suspended in ethyl acetate, collected by filtration, washed with ethyl acetate, and dried to obtain the title compound (3.24 g) as a yellow solid. The filtrate obtained by the filtration with ethyl acetate and the washing liquid obtained by the washing with ethyl acetate were combined, and purified by silica gel column chromatography (hexane/ethyl acetate) to obtain the title compound (511 mg) as a yellow solid.
¹H NMR (300 MHz, DMSO-d₆) δ 1.51 (9H, s), 7.63 (1H, dd, J=9.0, 2.2 Hz), 8.16 (1H, d, J=9.3 Hz), 8.47 (1H, d, J=2.0 Hz), 9.93 (1H, s), 10.06 (1H, s).

### Reference Example T013: production of tert-butyl (E)-(2-(2-bromovinyl)benzo[d]thiazol-6-yl)carbamate

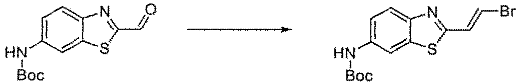

To a -78°C solution of (bromomethyl)triphenylphosphonium bromide (CAS [1034-49-7]) (4.23 g) in THF (50 ml), was added pottasium tert-butoxide (1M THF solution, 8.25 ml). The mixture was stirred at -78°C for 2 hours, followed by addition of a solution of tert-butyl (2-formylbenzo[d]thiazol-6-yl)carbamate (1.35 g) produced in Reference Example T012 in THF (15 ml). The mixture was stirred at -78°C for 1 hour, diluted at -78°C with ethyl acetate and 5% citric acid solution, neutralized at 0°C with 5% citric acid solution, diluted with water, and extracted with ethyl acetate. The extract was washed with water and brine, dried over anhydrous sodium sulfate, and concentrated under reduced pressure. The residue was purified by silica gel column chromatography (hexane/ethyl acetate) to obtain the title compound (374 mg) as a pale-yellow solid.
¹H NMR (300 MHz, DMSO-d₆) δ 1.50 (9H, s), 7.48 (1H, dd, J=9.0, 2.2 Hz), 7.55 (1H, d, J=14.0 Hz), 7.73 (1H, d, J=14.0 Hz), 7.86 (1H, d, J=8.9 Hz), 8.26 (1H, d, J=2.0 Hz), 9.68 (1H, s).

### Reference Example T014: production of tert-butyl (E)-(5-(4-(6-((tert-butoxycarbonyl)amino)benzo[d]thiazol-2-yl)but-3-en-1-yn-1-yl)pyrazin-2-yl(methyl)carbamate

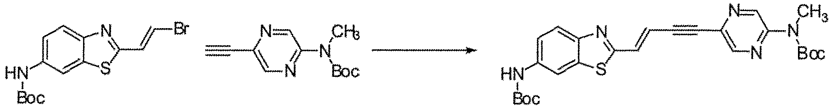

To a solution of tert-butyl (E)-(2-(2-bromovinyl)benzo[d]thiazol-6-yl)carbamate (370 mg) produced in Reference Example T013, tert-butyl (5-ethynylpyrazin-2-yl)(methyl)carbamate (267 mg) produced in Reference Example T005, and dichlorobis(triphenylphosphine)palladium (II) (37 mg) in THF (6 ml) and TEA (3 ml), was added copper iodide (I) (20 mg) at room temperature under nitrogen atmosphere. The mixture was stirred at room temperature for 2 hours, cooled to 0°C, diluted with ethyl acetate, adjusted to pH approximately 4 with 5% citric acid solution, and subjected to Celite filtration. The filtrate was extracted with ethyl acetate. The extract was washed with water and brine, dried over anhydrous sodium sulfate, passed through a short silica gel column, and concentrated under reduced pressure. The residue was purified by silica gel column chromatography (hexane/ethyl acetate) to obtain the title compound (436 mg) as a yellow solid.
¹H NMR (300 MHz, DMSO-d₆) δ 1.50 (9H, s), 1.51 (9H, s), 3.34 (3H, s), 7.01 (1H, d, J=16.1 Hz), 7.48 (1H, d, J=16.1 Hz), 7.50 (1H, dd, J=8.9, 2.1 Hz), 7.90 (1H, d, J=9.0 Hz), 8.30 (1H, d, J=2.0 Hz), 8.64 (1H, d, J=1.5 Hz), 9.06 (1H, d, J=1.5 Hz), 9.72 (1H, s).

### Reference Example T015: production of tert-butyl (E)-(5-(4-(6-((tert-butoxycarbonyl)amino)benzo[d]thiazol-2-yl)but-3-en-1-yn-1-yl)pyridin-2-yl(methyl)carbamate

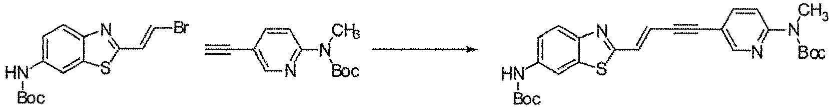

To a solution of tert-butyl (E)-(2-(2-bromovinyl)benzo[d]thiazol-6-yl)carbamate (100 mg) produced in Reference Example T013, tert-butyl (5-ethynylpyridin-2-yl)(methyl)carbamate (CAS [1565797-81-0]) (80 mg), and dichlorobis(triphenylphosphine)palladium (II) (9.9 mg) in THF (6 ml) and TEA (3 ml), was added copper iodide (I) (5.4 mg) at room temperature under nitrogen atmosphere. The mixture was stirred at room temperature for 2 hours, cooled to 0°C, diluted with ethyl acetate, adjusted to pH approximately 4 with 5% citric acid solution, and subjected to Celite filtration. The filtrate was extracted with ethyl acetate. The extract was washed with water and brine, dried over anhydrous sodium sulfate, passed through a short silica gel column, and concentrated under reduced pressure. The residue was purified by silica gel column chromatography (hexane/ethyl acetate) to obtain the title compound (140 mg) as a yellow solid.
¹H NMR (300 MHz, DMSO-d₆) δ 1.49 (9H, s), 1.50 (9H, s), 3.34 (3H, s), 6.96 (1H, d, J=16.1 Hz), 7.38 (1H, d, J=16.0 Hz), 7.46-7.54 (1H, m), 7.78 (1H, dd, J=8.7, 0.8 Hz), 7.89 (1H, brd, J=9.0 Hz), 7.90 (1H, dd, J=8.8, 2.3 Hz), 8.28 (1H, d, J=1.3 Hz), 8.55 (1H, dd, J=2.4, 0.8 Hz), 9.71 (1H, s).

### Reference Example T016: production of (E)-2-(4-(6-(methylamino) pyridin-3 -yl) bu t-1-en-3 -yn-1-yl)benzo[d]thiazol-6-amine

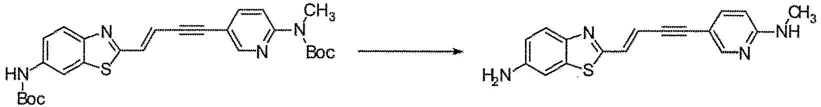

A mixture of tert-butyl (E)-(5-(4-(6-((tert-butoxycarbonyl)amino)benzo[d]thiazol-2-yl)but-3-en-1-yn-1-yl)pyridin-2-yl(methyl)carbamate (140 mg) produced in Reference Example T015, TFA (0.900 ml), and water (0.100 ml) was stirred at room temperature for 1 hour, cooled to 0°C, diluted with ethyl acetate, neutralized with a 5% sodium hydrogencarbonate solution, and extracted with ethyl acetate. The extract was washed with water and brine, dried over anhydrous sodium sulfate, passed through a short silica gel column, and concentrated under reduced pressure to obtain the title compound (84 mg) as a yellow solid.
¹H NMR (300 MHz, DMSO-d₆) δ 2.80 (3H, d, J=4.9 Hz), 5.58 (2H, s), 6.47 (1H, d, J=8.3 Hz), 6.71 (1H, d, J=15.8 Hz), 6.77 (1H, dd, J=8.7, 2.3 Hz), 7.03 (1H, d, J=2.3 Hz), 7.05-7.11 (1H, m), 7.08-7.16 (1H, m), 7.43-7.50 (1H, m), 7.61 (1H, d, J=8.7 Hz), 8.17 (1H, d, J=2.3 Hz).

### Reference Example T017: production of 2-hydroxy-3-iodopropyl 4-methylbenzenesulfonate

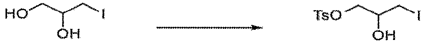

To a solution of 3-iodopropane-1,2-diol (CAS [554-10-9]) (3.40 g) in pyridine (12 ml), was added p-toluenesulfonyl chloride (3.21 g) at room temperature. The mixture was stirred at room temperature for 4 hours, cooled to 0°C, diluted with ethyl acetate and 1M hydrochloric acid, and extracted with ethyl acetate. The extract was washed with 1M hydrochloric acid, water, and brine, dried over anhydrous sodium sulfate, and concentrated under reduced pressure. The residue was purified by silica gel column chromatography (hexane/ethyl acetate) to obtain the title compound (3.95 g) as a colorless liquid.
¹H NMR (300 MHz, DMSO-d₆) δ 2.43 (3H, s), 3.14 (1H, dd, J=10.3, 5.6 Hz), 3.21 (1H, dd, J=10.3, 5.3 Hz), 3.57-3.69 (1H, m), 3.91 (1H, dd, J=9.9, 6.0 Hz), 3.97 (1H, dd, J=9.9, 4.4 Hz), 5.71 (1H, d, J=5.3 Hz), 7.50 (2H, dd, J=8.6, 0.7 Hz), 7.80 (2H, d, J=8.3 Hz).

### Reference Example T018: production of 3-iodo-2-((tetrahydro-2H-pyran-2-yl)oxy)propyl 4-methylbenzenesulfonate

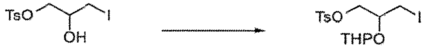

To a solution of 2-hydroxy-3-iodopropyl 4-methylbenzenesulfonate (2.00 g) produced in Reference Example T017 and 3,4-dihydro-2H-pyrane (5.12 ml) in THF (25 ml), was added pyridinium p-toluenesulfonate (1.41 g) at room temperature. The mixture was stirred at room temperature for 4 hours, followed by addition of p-toluenesulfonic acid monohydrate (534 mg) at room temperature. The mixture was stirred at room temperature for 4 hours, cooled to 0°C, diluted with ethyl acetate, neutralized with a 5% sodium hydrogencarbonate solution, diluted with water, and extracted with ethyl acetate. The extract was washed with water and brine, dried over anhydrous sodium sulfate, and concentrated under reduced pressure. The residue was purified by silica gel column chromatography (hexane/ethyl acetate) to obtain the title compound (2.09 g) as a colorless liquid.
¹H NMR (300 MHz, DMSO-d₆) δ 1.28-1.73 (6H, m), 2.43 (3H, s), 3.27-3.47 (3H, m), 3.56-3.89 (2H, m), 4.04-4.20 (2H, m), 4.59-4.78 (1H, m), 7.50 (2H, d, J=8.0 Hz), 7.81 (2H, dd, J=8.3, 1.3 Hz).

### Reference Example T019: production of 3-hydroxy-2,2-dimethoxypropyl 4-methylbenzenesulfonate

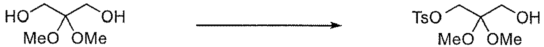

To a solution of 2,2-dimethoxypropane-1,3-diol (CAS [153214-82-5]) (2.28 g) in pyridine (6 ml) was slowly added p-toluenesulfonyl chloride (1.00 g) at 0°C. The mixture was stirred at 0°C to room temperature for 3 days, cooled to 0°C, diluted with ethyl acetate, water, and 1M hydrochloric acid, and extracted with ethyl acetate. The extract was washed with water and brine, dried over anhydrous sodium sulfate, and concentrated under reduced pressure. The residue was purified by silica gel column chromatography (hexane/ethyl acetate) to obtain the title compound (389 mg) as a white solid.
¹H NMR (300 MHz, DMSO-d₆) δ 2.43 (3H, s), 3.04 (6H, s), 3.32 (2H, brd, J=5.5 Hz), 3.89 (2H, s), 4.92 (1H, t, J=5.6 Hz), 7.49 (2H, d, J=8.0 Hz), 7.82 (2H, d, J=8.3 Hz).

### Reference Example T020: production of 2,2-dimethoxy-3-oxopropyl 4-methylbenzenesulfonate

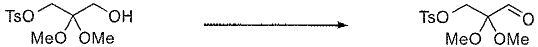

To a solution of 3-hydroxy-2,2-dimethoxypropyl 4-methylbenzenesulfonate (389 mg) produced in Reference Example T019 in acetonitrile (20 ml), was added 1,1,1-triacetoxy-1,1-dihydro-1,2-benziodoxol-3-(1H)-one (1.14 g) at room temperature. The mixture was stirred at room temperature for 2 hours, cooled to 0°C, diluted with ethyl acetate and saturated sodium thiosulfate solution, neutralized with a 5% sodium hydrogencarbonate solution, and extracted with ethyl acetate. The extract was washed with a 5% sodium hydrogencarbonate solution, water, and brine in this order, dried over anhydrous sodium sulfate, and concentrated under reduced pressure. The residue was purified by silica gel column chromatography (hexane/ethyl acetate) to obtain the title compound (336 mg) as a colorless liquid.
¹H NMR (300 MHz, DMSO-d₆) δ 2.43 (3H, s), 3.18 (6H, s), 4.14 (2H, s), 7.50 (2H, dd, J=8.6, 0.7 Hz), 7.79 (2H, d, J=8.3 Hz), 9.44 (1H, s).

### Reference Example T021: production of (E)-2-(4-(5-(methylamino) pyrazin-2-yl)but-1-en-3-yn-1-yl)benzo [d]thiazol-6-amine

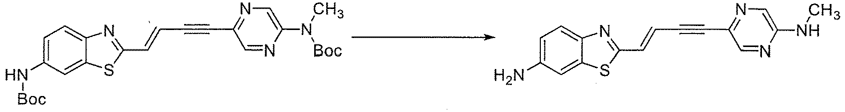

A mixture of tert-butyl (E)-(5-(4-(6-((tert-butoxycarbonyl) amino) benzo[d]thiazol-2-yl)but-3-en-1-yn-1-yl)pyrazin-2-yl(methyl)carbamate (736 mg) produced in Reference Example T014, TFA (2.70 ml), and water (0.300 ml) was stirred at room temperature for 1 hour, cooled to 0°C, diluted with ethyl acetate, neutralized with a 5% sodium hydrogencarbonate solution, and extracted with a mixed solvent of ethyl acetate and THF. The extract was washed with water and brine, dried over anhydrous sodium sulfate, and concentrated under reduced pressure to obtain the title compound (446 mg) as a yellow solid.
¹H NMR (300 MHz, DMSO-d₆) δ 2.84 (3H, d, J=4.9 Hz), 5.60 (2H, s), 6.74 (1H, d, J=16.1 Hz), 6.74-6.83 (1H, m), 7.04 (1H, d, J=2.2 Hz), 7.20 (1H, d, J=16.0 Hz), 7.57-7.64 (1H, m), 7.63 (1H, d, J=8.8 Hz), 7.93 (1H, d, J=1.4 Hz), 8.19 (1H, d, J=1.3 Hz).

### Reference Example T022: production of (E)-2,2-dimethoxy-3-((2-((E)-(4-(5-(methylamino)pyrazin-2-yl)but-1-en-3-yn-1-yl)benzo [d] thiazol-6-yl)imino) propyl 4-methylbenzenesulfonate

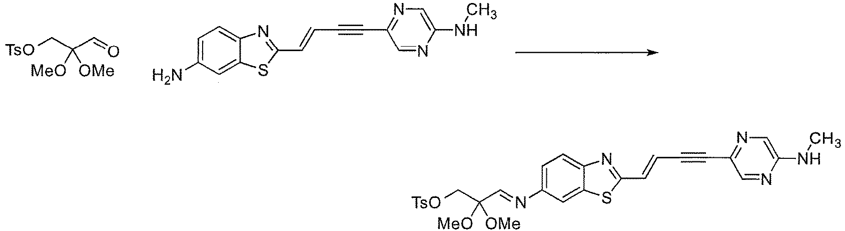

A suspension of 2,2-dimethoxy-3-oxopropyl 4-methylbenzenesulfonate (560 mg) produced in Reference Example T020 and (E)-2-(4-(5-(methylamino)pyrazin-2-yl)but-1-en-3-yn-1-yl)benzo[d]thiazol-6-amine (552 mg) produced in Reference Example T021 in ethyl acetate (60 ml) and THF (20 ml) was stirred at room temperature for 2 hours, followed by further addition of THF (80 ml). The resulting solution was stirred at 40°C for 2 hours and stirred at room temperature overnight. After the stirring, the solution was concentrated under reduced pressure to obtain the title compound (1.05 g) as a yellow solid.
¹H NMR (300 MHz, DMSO-d₆) δ 2.30 (3H, s), 2.85 (3H, d, J=4.9 Hz), 3.24 (6H, s), 4.29 (2H, s), 7.07 (1H, d, J=16.0 Hz), 7.20 (1H, dd, J=8.7, 2.2 Hz), 7.36 (1H, d, J=16.0 Hz), 7.36 (2H, d, J=7.9 Hz), 7.69 (1H, q, J=4.9 Hz), 7.75-7.79 (3H, m), 7.87 (1H, s), 7.95 (1H, d, J=1.5 Hz), 7.97 (1H, d, J=8.9 Hz), 8.23 (1H, d, J=1.4 Hz).

### Reference Example T023: production of (E)-2,2-dimethoxy-3-((2-(4-(5-(methylamino)pyrazin-2-yl)but-1-en-3-yn-1-yl)benzo[d]thiazol-6-yl)amino)propyl 4-methylbenzenesulfonate

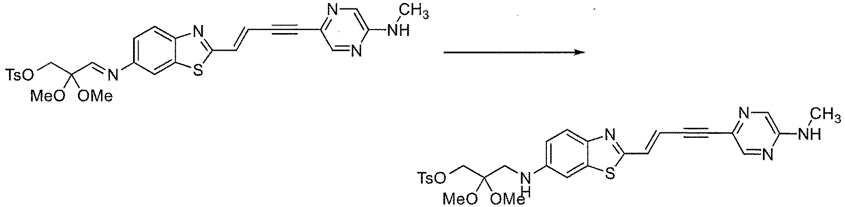

To a suspension of (E)-2,2-dimethoxy-3-((2-((E)-(4-(5-(methylamino) pyrazin-2-yl)but-1-en-3-yn-1-yl)benzo [d] thiazol-6-yl)imino) propyl 4-methylbenzenesulfonate (1.05 g) produced in Reference Example T022 in THF (40 ml), was added a solution of sodium boron hydride (69 mg) in DMSO (10 ml) and was then added EtOH (20 ml) at 0°C. The mixture was stirred at 0°C for 1 hour, diluted with ethyl acetate and water, adjusted to pH approximately 4 with 5% citric acid solution, and extracted with ethyl acetate. The extract was washed with water and brine, dried over anhydrous sodium sulfate, and concentrated under reduced pressure. The residue was purified by silica gel column chromatography (hexane/ethyl acetate) to obtain the title compound (935 mg) as a yellow solid.
¹H NMR (300 MHz, DMSO-d₆) δ 2.20 (3H, s), 2.84 (3H, d, J=4.8 Hz), 3.12-3.16 (2H, m), 3.14 (6H, s), 4.02 (2H, s), 5.73 (1H, t, J=5.1 Hz), 6.80 (1H, d, J=16.1 Hz), 6.85 (1H, dd, J=9.0, 2.2 Hz), 7.01 (1H, d, J=2.3 Hz), 7.16-7.27 (3H, m), 7.61-7.70 (4H, m), 7.94 (1H, d, J=1.5 Hz), 8.20 (1H, d, J=1.4 Hz).

### Reference Example T024: production of (E)-3-((2-(4-(5-(methylamino) pyrazin-2-yl)but-1-en-3-yn-1-yl)benzo [d] thiazol-6-yl)amino)-2-oxopropyl 4-methylbenzenesulfonate

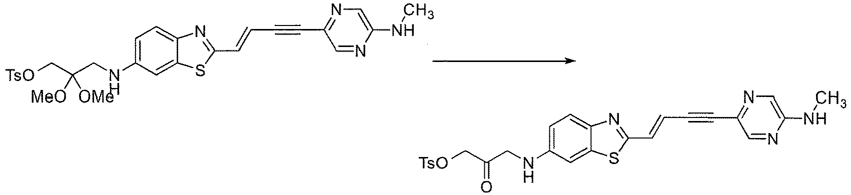

A mixture of (E)-2,2-dimethoxy-3-((2-(4-(5-(methylamino)pyrazin-2-yl)but-1-en-3-yn-1-yl)benzo [d] thiazol-6-yl)amino) propyl 4-methylbenzenesulfonate (132 mg) produced in Reference Example T023, TFA (1.35 ml), and water (0.15 ml) was stirred at room temperature for 1 hour, cooled to 0°C, diluted with ethyl acetate, neutralized with a 5% sodium hydrogencarbonate solution, and extracted with ethyl acetate. The extract was washed with water and brine, dried over anhydrous sodium sulfate, passed through a short silica gel column, and concentrated under reduced pressure. The residual solid was suspended in ethyl acetate, collected by filtration, washed with ethyl acetate, and dried to obtain the title compound (83 mg) as an orange solid.
¹H NMR (300 MHz, DMSO-d₆) δ 2.41 (3H, s), 2.84 (3H, d, J=4.9 Hz), 4.11 (2H, d, J=5.9 Hz), 5.01 (2H, s), 6.45 (1H, t, J=5.7 Hz), 6.79 (1H, d, J=16.0 Hz), 6.85 (1H, dd, J=9.0, 2.4 Hz), 6.96 (1H, d, J=2.3 Hz), 7.22 (1H, d, J=16.0 Hz), 7.47 (2H, d, J=7.9 Hz), 7.62 (1H, q, J=6.0 Hz), 7.67 (1H, d, J=8.9 Hz), 7.80 (2H, d, J=8.4 Hz), 7.93 (1H, d, J=1.4 Hz), 8.19 (1H, d, J=1.3 Hz).

### Reference Example T025: production of (E)-2-hydroxy-3-((2-(4-(5-(methylamino)pyrazin-2-yl)but-1-en-3-yn-1-yl)benzo [d] thiazol-6-yl)amino) propyl 4-methylbenzenesulfonate

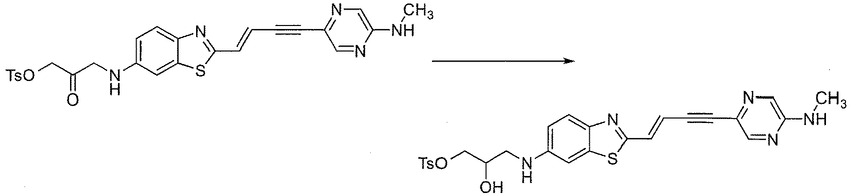

To a suspension of (E)-3-((2-(4-(5-(methylamino)pyrazin-2-yl)but-1-en-3-yn-1-yl)benzo [d] thiazol-6-yl)amino)-2-oxopropyl 4-methylbenzenesulfonate (83 mg) produced in Reference Example T024 in EtOH (20 ml) and THF (20 ml), was added sodium boron hydride (8.8 mg) at room temperature. The suspension was subjected to ultrasonication to obtain a solution, followed by stirring at room temperature for 10 hours. After the stirring, the mixture was cooled to 0°C, diluted with ethyl acetate and water, acidified to pH approximately 4 with 5% citric acid solution, and extracted with ethyl acetate. The extract was washed with water and brine, dried over anhydrous sodium sulfate, and concentrated under reduced pressure. The residue was purified by silica gel column chromatography (hexane/ethyl acetate) to obtain the title compound (50 mg) as a yellow solid.
¹H NMR (300 MHz, DMSO-d₆) δ 2.36 (3H, s), 2.84 (3H, d, J=4.9 Hz), 3.00-3.17 (2H, m), 3.76-3.89 (1H, m), 3.96 (1H, dd, J=10.1, 6.3 Hz), 4.08 (1H, dd, J=9.7, 3.7 Hz), 5.37 (1H, d, J=5.1 Hz), 6.13 (1H, t, J=6.4 Hz), 6.78 (1H, d, J=16.1 Hz), 6.81 (1H, dd, J=8.6, 2.6 Hz), 7.01 (1H, d, J=2.1 Hz), 7.21 (1H, d, J=16.0 Hz), 7.40 (2H, d, J=7.9 Hz), 7.62 (1H, q, J=5.5 Hz), 7.65 (1H, d, J=9.0 Hz), 7.76 (2H, d, J=8.3 Hz), 7.93 (1H, d, J=1.4 Hz), 8.19 (1H, d, J=1.3 Hz).

### Example 1: production of (E)-1-fluoro-3-((2-(4-(5-methylamino)pyrazin-2-yl)but-1-en-3-yn-1-yl)benzo[d]thiazol-6-yl)amino)propan-2-ol (compound (I); hereinafter also referred to as "SPAL-T-83")

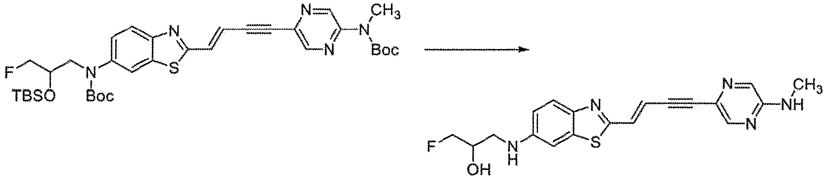

A mixture of tert-butyl (E)-(2-(4-(5-((tert-butoxycarbonyl)(methyl)amino)pyrazin-2-yl)but-1-en-3-yn-1-yl)benzo[d]thiazol-6-yl)(2-((tert-butyldimethylsilyl)oxy)-3-fluoropropyl)carbamate (22 mg) produced in Reference Example T011, TFA (0.900 ml), and water (0.100 ml) was stirred at room temperature for 4 hours, cooled to 0°C, diluted with ethyl acetate, neutralized with a 5% sodium hydrogencarbonate solution, and extracted with ethyl acetate. The extract was washed with water and brine, dried over anhydrous sodium sulfate, passed through a short silica gel column, and concentrated under reduced pressure. The residual solid was suspended in ethyl acetate, collected by filtration, washed with ethyl acetate, and dried to obtain the title compound (11.5 mg) as an orange solid.
¹H NMR (300 MHz, DMSO-d₆) δ 2.84 (3H, d, J=4.9 Hz), 3.02-3.15 (1H, m), 3.16-3.28 (1H, m), 3.81-3.98 (1H, m), 4.28-4.58 (2H, m), 5.29 (1H, d, J=5.3 Hz), 6.20 (1H, t, J=6.0 Hz), 6.76 (1H, d, J=15.8 Hz), 6.88 (1H, dd, J=9.0, 2.3 Hz), 7.10 (1H, d, J=2.3 Hz), 7.21 (1H, d, J=16.2 Hz), 7.61 (1H, q, J=4.9 Hz), 7.67 (1H, d, J=9.0 Hz), 7.93 (1H, d, J=1.5 Hz), 8.19 (1H, d, J=1.5 Hz)

### Example 2: production of (E)-1-fluoro-3-((2-(4-(6-methylamino)pyridin-3-yl)but-1-en-3-yn-1-yl)benzo[d]thiazol-6-yl)amino)propan-2-ol (compound (II); hereinafter also referred to as "SPAL-T-95")

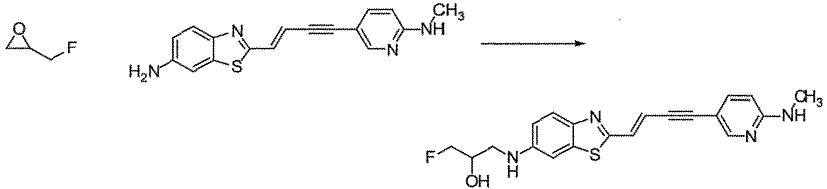

A mixture of (E)-2-(4-(6-(methylamino)pyridin-3-yl)but-1-en-3-yn-1-yl)benzo[d]thiazol-6-amine (40 mg) produced in Reference Example T016, 2-(fluoromethyl)oxirane (CAS [503-09-3]) (0.047 ml), and methoxyethane-1-ol (6 ml) was stirred at 150°C for 1 hour under microwave irradiation. The reaction mixture was cooled to room temperature, followed by addition of 2-(fluoromethyl)oxirane (0.047 ml). The mixture was stirred at 150°C for 1 hour under microwave irradiation, cooled to room temperature, and concentrated under reduced pressure. The residue was purified by silica gel column chromatography (hexane/ethyl acetate) to obtain the title compound (7.0 mg) as a yellow solid.
¹H NMR (300 MHz, DMSO-d₆) δ 2.80 (3H, d, J=4.9 Hz), 3.01-3.28 (2H, m), 3.81-3.97 (1H, m), 4.28-4.58 (2H, m), 5.30 (1H, d, J=5.0 Hz), 6.18 (1H, t, J=6.1 Hz), 6.47 (1H, d, J=8.7 Hz), 6.73 (1H, d, J=16.0 Hz), 6.87 (1H, dd, J=8.8, 2.3 Hz), 7.03-7.14 (2H, m), 7.12 (1H, d, J=16.0 Hz), 7.47 (1H, dd, J=8.8, 2.4 Hz), 7.65 (1H, d, J=8.9 Hz), 8.17 (1H, d, J=2.3 Hz).

### Example 3: production of (E)-3((tert-butoxycarbonyl)(2-(4-(5-((tert-butoxycarbonyl) (methyl)amino)pyrazin-2-yl)but- 1 - en-3-yn-1-yl)benzo[d]thiazol-6-yl)amino)-2-((tetrahydro-2H-pyran-2-yl)oxy)propyl 4-methylbenzenesulfonate

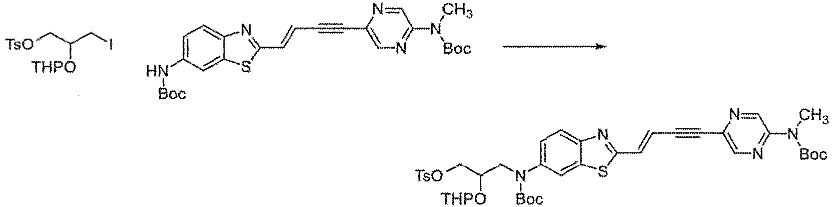

To a 0°C solution of tert-butyl (E)-(5-(4-(6-((tert-butoxycarbonyl)amino)benzo[d]thiazol-2-yl)but-3-en-1-yn-1-yl)pyrazin-2-yl(methyl)carbamate (436 mg) produced in Reference Example T014 in DMF (20 ml), was added sodium hydride (60% in oil, 41 mg). The mixture was stirred at 0°C for 5 minutes, followed by addition of a solution of 3-iodo-2-((tetrahydro-2H-pyran-2-yl)oxy)propyl 4-methylbenzenesulfonate (567 mg) produced in Reference Example T018 in DMF (5 ml). The mixture was stirred at room temperature for 2 hours, cooled to 0°C, diluted with ethyl acetate and water, neutralized with 5% citric acid solution, and extracted with ethyl acetate. The extract was washed with water and brine, dried over anhydrous sodium sulfate, and concentrated under reduced pressure. The residue was purified by silica gel column chromatography (hexane/ethyl acetate) to obtain the title compound (42 mg) as a yellow solid. ¹H NMR (300 MHz, DMSO-d₆) δ 1.23-1.43 (6H, m), 1.37 (9H, s), 1.52 (9H, s), 2.41 (3H, s), 3.18-3.28 (1H, m), 3.34 (3H, s), 3.51-3.64 (1H, m), 3.69-3.85 (2H, m), 3.85-3.96 (1H, m), 3.98-4.10 (2H, m), 4.50 (1H, brs), 7.12 (1H, d, J=16.1 Hz), 7.30-7.47 (1H, m), 7.43 (2H, brd, J=8.1 Hz), 7.54 (1H, d, J=16.1 Hz), 7.66-7.79 (2H, m), 7.94 (1H, d, J=8.7 Hz), 7.98-8.05 (1H, m), 8.66 (1H, d, J=1.4 Hz), 9.07 (1H, d, J=1.3 Hz).

### Example 4: production of (E)-3-(2-(4-(5-(methylamino) pyrazin-2-yl)but-1-en-3-yn-1-yl)benzo[d]thiazol-6-yl)oxazolidine-5-yl)methyl 4-methylbenzenesulfonate

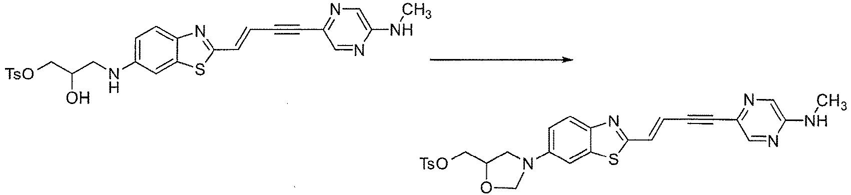

A mixture of (E)-2-hydroxy-3-((2-(4-(5-(methylamino)pyrazin-2-yl)but-1 -en-3-yn- 1 - yl)benzo [d] thiazol-6-yl)amino) propyl 4-methylbenzenesulfonate (375 mg) produced in Reference Example T025 and paraformaldehyde (63 mg) in acetonitrile (80 ml) was stirred at 60°C for 0.5 hours, followed by addition of paraformaldehyde (42 mg). The mixture was further stirred at 60°C for 0.5 hours, cooled to room temperature, passed through a short silica gel column to elute with ethyl acetate, and concentrated under reduced pressure. The residue was purified by silica gel column chromatography (hexane/ethyl acetate) to obtain the title compound (166 mg) as a yellow solid.
¹H NMR (300 MHz, DMSO-d₆) δ 2.42 (3H, s), 2.84 (3H, d, J=4.8 Hz), 3.19 (1H, dd, J=9.2, 6.5 Hz), 3.55 (1H, dd, J=9.4, 7.3 Hz), 4.19 (1H, dd, J=11.0, 6.1 Hz), 4.24 (1H, dd, J=10.8, 3.5 Hz), 4.49-4.61 (1H, m), 4.84 (1H, d, J=2.8 Hz), 4.89 (1H, d, J=2.8 Hz), 6.77 (1H, dd, J=9.1, 2.4 Hz), 6.85 (1H, d, J=16.1 Hz), 7.13 (1H, d, J=2.4 Hz), 7.25 (1H, d, J=16.1 Hz), 7.48 (2H, d, J=7.9 Hz), 7.64 (1H, q, J=4.6 Hz), 7.77-7.85 (3H, m), 7.94 (1H, d, J=1.5 Hz), 8.20 (1H, d, J=1.4 Hz).

### Reference Example E001: production of di-tert-butyl (5-iodopyrazin-2-yl)-2-imidodicarbonate

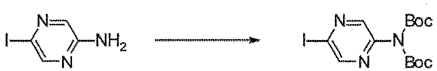

To a solution of 5-iodopyrazin-2-amine (CAS [886860-50-0]) (2.86 g) in THF (100 ml), were added di-tert-butyl dicarbonate (7.06 g) and N,N-dimethylpyridin-4-amine (0.316 g) at room temperature. The mixture was stirred at room temperature for 3 days. Water and ethyl acetate were added to the reaction solution, and the aqueous layer was extracted with ethyl acetate. The organic layers were combined, washed with water and saturated brine, dried over anhydrous sodium sulfate, and filtered. The filtrate was distilled under reduced pressure. The residue thus obtained was purified by column chromatography (silica gel, 40%-60% ethyl acetate/n-heptane) to obtain the title compound (4.8375 g).
¹H NMR (400 MHz, CDCl₃) δ (ppm): 8.65-8.70 (m, 1H), 8.35-8.39 (m, 1H), 1.43 (s, 18H).
MS (ESI) m/z: 422 [M+H]⁺.

### Reference Example E002: production of tert-butyl (5-iodopyrazin-2-yl)carbamate

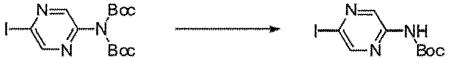

To a solution of di-tert-butyl (5-iodopyrazin-2-yl)-2-imidodicarbonate (4.8375 g) produced in Reference Example E001 in methanol (50 ml), was added potassium carbonate (4.76 g) at room temperature. The mixture was stirred at room temperature for 6 hours and then stirred at 70°C for 1 hour. After the reaction solution had been cooled to room temperature, water and ethyl acetate were added thereto, and the aqueous layer was extracted with ethyl acetate. The organic layers were combined, washed with water and saturated brine, dried over sodium sulfate, and filtered. The filtrate was distilled under reduced pressure to obtain the title compound (3.5023 g).
¹H-NMR (400 MHz, CDCl₃) δ (ppm): 1.54 (s, 9H), 3.84 (s, 1H), 8.44 (d, 1H, J=1.8 Hz), 9.12 (d, 1H, J=1.4 Hz).
MS (ESI) m/z: 322 [M+H]⁺.

### Reference Example E003: production of tert-butyl (5-iodopyrazin-2-yl)(methyl)carbamate

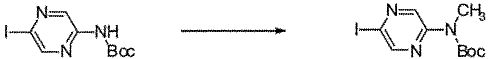

To a solution of tert-butyl (5-iodopyrazin-2-yl)carbamate (3.5023 g) produced in Reference Example E002 in DMF (55 ml), were added cesium carbonate (7.11 g) and iodomethane (1.018 ml) at 0°C. The mixture was stirred at room temperature for 19 hours. Water and ethyl acetate were added to the reaction solution, and the aqueous layer was extracted with ethyl acetate. The organic layers were combined, washed with water and saturated brine, dried over anhydrous sodium sulfate, and filtered. The filtrate was distilled under reduced pressure. The residue thus obtained was purified by column chromatography (silica gel, 0%-30% ethyl acetate/n-heptane) to obtain the title compound (3.2794 g).
¹H-NMR (400 MHz, CDCl₃) δ (ppm): 1.53 (s, 9H), 3.34 (s, 3H), 8.51 (d, 1H, J=1.4 Hz), 8.93 (d, 1H, J=1.4 Hz).
MS (ESI) m/z: 336 [M+H]⁺.

### Reference Example E004: production of tert-butyl (5-(3-hydroxyprop-1-yn-1-yl)pyrazin-2-yl)(methyl)carbamate

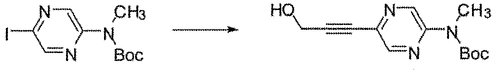

To a suspension of tert-butyl (5-iodopyrazin-2-yl)(methyl)carbamate (3.2794 g) produced in Reference Example E003 in triethylamine (9.55 ml), were added propargyl alcohol (1.129 ml), copper iodide (I) (0.224 g), and bis(triphenylphosphine)palladium (II) chloride (0.137 g). The mixture was stirred at room temperature for 90 minutes under nitrogen atmosphere. Water was added to the reaction solution, and the aqueous layer was extracted with ethyl acetate. The organic layers were combined, dried over anhydrous magnesium sulfate, and filtered. The filtrate was distilled under reduced pressure. The residue thus obtained was purified by column chromatography (silica gel, 30%-50% ethyl acetate/n-heptane) to obtain the title compound (2.1609 g).
¹H-NMR (400 MHz, CDCl₃) δ (ppm): 1.52 (s, 9H), 3.36 (s, 3H), 4.52 (s, 2H), 8.37 (d, 1H, J=1.4 Hz), 9.06 (s, 1H). MS (ESI) m/z: 264 [M+H]⁺.

### Reference Example E005: production of tert-butyl methyl(5-(3-oxoprop-1-yn-1-yl)pyrazin-2-yl)carbamate

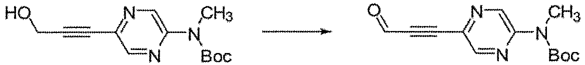

To a solution of oxalyl chloride (1.409 ml) in dichloromethane (40 ml) was slowly added a solution of DMSO (1.747 ml) in dichloromethane (10 ml) at -78°C. The mixture was stirred for 5 minutes. To the reaction solution was slowly added a solution of tert-butyl (5-(3-hydroxyprop-1-yn-1-yl)pyrazin-2-yl)(methyl)carbamate (2.1609 g) produced in Reference Example E004 in dichloromethane (20 ml) at -78°C. The mixture was stirred at -78°C for 1 hour. To the reaction solution was added triethylamine (6.86 ml) at -78°C. The mixture was stirred at -78°C for 30 minutes. The reaction solution was raised in temperature to room temperature, a saturated ammonium chloride solution and saturated brine were added thereto, and the aqueous layer was extracted with ethyl acetate. The organic layers were combined, dried over anhydrous sodium sulfate, and filtered. The filtrate was distilled under reduced pressure. The residue thus obtained was purified by column chromatography (silica gel, 30%-50% ethyl acetate/n-heptane) to obtain the title compound (1.4440 g).
¹H-NMR (400 MHz, CDCl₃) δ (ppm): 1.49 (d, 9H, J=1.8 Hz), 3.34-3.37 (m, 3H), 8.49 (d, 1H, J=1.4 Hz), 9.23 (d, 1H, J=1.4 Hz), 9.36-9.40 (m, 1H).
MS (ESI) m/z: 262 [M+H]⁺.

### Reference Example E006: production of 5-chloro-2-methylthiazolo[5,4-b]pyridine

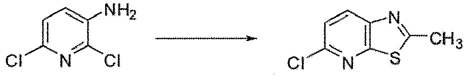

Sodium carbonate (7.26 g), 3-amino-2,6-dichloropyridine (CAS: 62476-56-6, 4.29 g), and thiophosgene (2.62 ml) were suspended in dichloromethane (50 ml), and the mixture was stirred at room temperature for 2 days. The mixture was subjected to Celite (registered trademark) filtration to remove the insoluble, and washed with dichloromethane. The filtrate was distilled under reduced pressure to obtain a 2,6-dichloro-3-isothiocyanatepyridine intermediate. This intermediate was dissolved in THF (12.5 ml). The solution was slowly added dropwise to a 3M THF solution (12.29 ml) of methyl magnesium chloride at 0°C under nitrogen atmosphere. The mixture was stirred at 0°C for 2 hours. After a saturated ammonium chloride solution had been slowly added to the mixture at 0°C, the mixture was raised in temperature to room temperature. Ethyl acetate was added, and the aqueous layer was extracted with ethyl acetate. The organic layers were combined, dried over anhydrous sodium sulfate, and filtered. The filtrate was distilled under reduced pressure to obtain a N-(2,6-dichloropyridin-3-yl)ethanethioamide intermediate. This intermediate was dissolved in DMF (25 ml). To the solution was added sodium carbonate (4.19 g). The mixture was stirred at 90°C for 1 hour under nitrogen atmosphere. The mixture was poured into ice water. The solution was strongly stirred for 30 minutes. The produced solid was filtered out, washed with water, and dried under reduced pressure to obtain the title compound (4.84 g).
¹H-NMR (400 MHz, CDCl₃) δ (ppm): 2.84 (s, 3H), 7.39 (d, J=8.6 Hz, 1H), 8.09 (d, J=8.6 Hz, 1H).
MS (ESI) m/z: 185 [M+H]⁺.

### Reference Example E007: production 1 of 2-(bromomethyl)-5-chlorothiazolo[5,4-b]pyridine

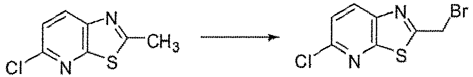

To a solution of 5-chloro-2-methylthiazolo[5,4-b]pyridine (CAS: 109202-21-3, 500 mg) produced in Reference Example E006 in alpha, alpha, alpha-trifluorotoluene (18 ml) were added N-bromosuccinimide (723 mg) and 2,2'-azobis(isobutyronitrile) (89 mg). The mixture was stirred at 80°C for 2 hours. To the mixture was added 2,2'-azobis(isobutyronitrile) (35.6 mg). The mixture was stirred at 80°C for 2 hours. After the mixture had been cooled to room temperature, water and ethyl acetate were added thereto, and the aqueous layer was extracted with ethyl acetate. The organic layers were combined, dried over anhydrous sodium sulfate and filtered. The filtrate was distilled under reduced pressure. The residue thus obtained was purified by column chromatography (silica gel, 0%-30% ethyl acetate/n-heptane) to obtain the title compound (319 mg). ¹H-NMR (400 MHz, CDCl₃) δ (ppm): 4.76 (d, J=2.3 Hz, 2H), 7.40-7.47 (m, 1H), 8.10-8.23 (m, 1H).
MS (ESI) m/z: 263, 265 [M+H]⁺.

### Reference Example E008: production of diethyl ((5-chlorothiazolo[5,4-b]pyridin-2-yl)methyl) phos phonate

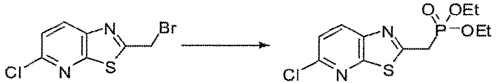

A mixture of 2-(bromomethyl)-5-chlorothiazolo[5,4-b]pyridine (319 mg) produced in Reference Example E007 and triethyl phosphite (15 ml) was stirred at 100°C for 2 hours. The mixture was cooled to room temperature, followed by distillation under reduced pressure. The residue thus obtained was purified by column chromatography (silica gel, 0%-100% ethyl acetate/n-heptane, 5% methanol/ethyl acetate) to obtain the title compound (305 mg).
¹H-NMR (400 MHz, CDCl₃) δ (ppm): 1.30 (dt, J=0.9, 7.0 Hz, 6H), 3.64-3.74 (m, 2H), 4.15 (q, J=7.4 Hz, 4H), 7.40 (dd, J=1.4, 8.6 Hz, 1H), 8.14 (dd, J=0.9, 8.6 Hz, 1H).
MS (ESI) m/z: 321 [M+H]⁺.

### Reference Example E009: production of diethyl ((5-((3,4-dimethoxybenzyl)oxy)thiazolo[5,4-b]pyridin-2-yl)methyl) phosphonate

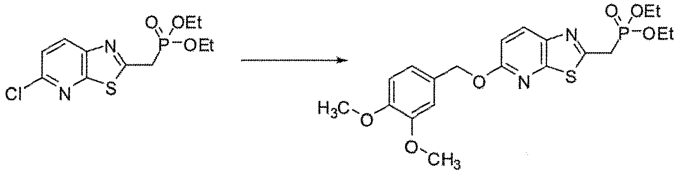

A mixture of diethyl ((5-chlorothiazolo[5,4-b]pyridin-2-yl)methyl)phosphonate (50 mg) produced in Reference Example E008, 3,4-dimethoxybenzyl alcohol (27.2 µl), (R)-(-)-1-[(S)-2-(dicyclohexylphosphino)ferrocenyl]ethyldi-tert-butylphosphine (12.97 mg), cesium carbonate (102 mg), and palladium (π-cinnamyl) chloride dimer (4.04 mg) in toluene (0.45 ml) was stirred at room temperature for 20 hours under nitrogen atmosphere. The mixture was directly purified by column chromatography (silica gel, 0%-100% ethyl acetate/n-heptane, 5% methanol/ethyl acetate) to obtain the title compound (33.1 mg).
¹H-NMR (400 MHz, CDCl₃) δ (ppm): 1.32 (t, J=7.0 Hz, 6H), 3.61-3.71 (m, 2H), 3.89 (d, J=6.3 Hz, 6H), 4.16 (q, J=7.5 Hz, 4H), 5.35 (s, 2H), 6.83-6.91 (m, 2H), 7.02 (s, 2H), 8.07 (d, J=8.6 Hz, 1H).
MS (ESI) m/z: 453 [M+H]⁺.

### Reference Example E010: production of tert-butyl (E)-(5-(4-(5-((3,4-dimethoxybenzyl)oxy)thiazolo[5,4-b]pyridin-2-yl)but-3-en-1-yn-1-yl)pyrazin-2-yl)(methyl)carbamate

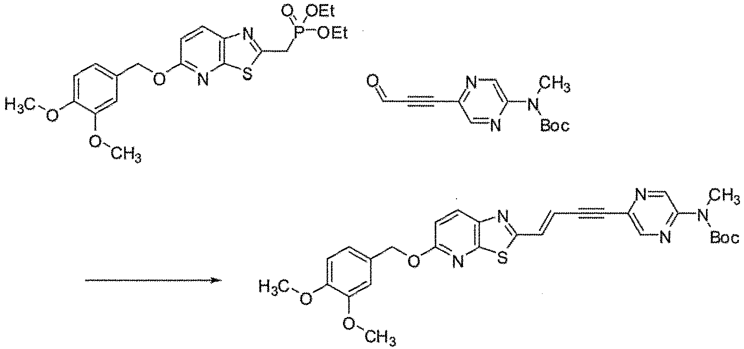

To a solution of diethyl ((5-((3,4-dimethoxybenzyl)oxy)thiazolo[5,4-b]pyridin-2-yl)methyl)phosphonate (33 mg) produced in Reference Example E009 in THF (1.5 ml), was added sodium hydride (4.38 mg, 60% oil dispersion) at 0°C. The mixture was stirred at 0°C for 30 minutes. To the mixture was added a solution of tert-butyl methyl(5-(3-oxoprop-1-yn-1-yl)pyrazin-2-yl)carbamate (38.1 mg) produced in Reference Example E005 in THF (0.75 ml) at 0°C. The mixture was stirred at 0°C for 1 hour. A saturated ammonium chloride solution was added to the mixture, and the aqueous layer was extracted with ethyl acetate. The organic layers were combined, dried over anhydrous magnesium sulfate, and filtered. The filtrate was distilled under reduced pressure. The residue thus obtained was purified by column chromatography (silica gel, 0%-30% ethyl acetate/n-heptane) to obtain the title compound (28.6 mg).
¹H-NMR (400 MHz, CDCl₃) δ (ppm): 1.55 (s, 9H), 3.39-3.42 (m, 3H), 3.86-3.91 (m, 6H), 5.37 (s, 2H), 6.78 (d, J=15.9 Hz, 1H), 6.85-6.92 (m, 2H), 7.00-7.07 (m, 2H), 7.27 (d, J=16.3 Hz, 1H), 8.09 (d, J=9.1 Hz, 1H), 8.45 (d, J=1.4 Hz, 1H), 9.15 (d, J=1.4 Hz, 1H).
MS (ESI) m/z: 560 [M+H]⁺.

### Reference Example E011: production of (E)-2-(4-(5-(methylamino)pyrazin-2-yl)but-1-en-3-yn-1-yl)thiazolo[5,4-b]pyridin-5-ol

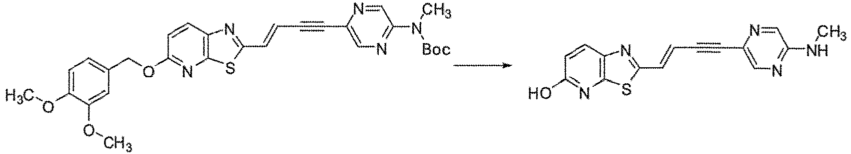

To a solution of tert-butyl (E)-(5-(4-(5-((3,4-dimethoxybenzyl)oxy)thiazolo[5,4-b]pyridin-2-yl)but-3-en-1-yn-1-yl)pyrazin-2-yl)(methyl)carbamate (28.6 mg) produced in Reference Example E010 in dichloromethane (0.8 ml), was added trifluoroacetic acid (0.5 ml) at 0°C. The mixture was stirred at room temperature for 4 hours. The mixture was exposed to reduced pressure to obtain the unpurified title compound (32.8 mg).
¹H-NMR (400 MHz, DMSO-d₆) δ (ppm): 2.79 (s, 3H), 6.71 (brd, J=9.1 Hz, 1H), 6.86 (brd, J=16.3 Hz, 1H), 7.19 (d, J=15.9 Hz, 1H), 7.91 (s, 1H), 8.09 (brd, J=9.1, 1H), 8.14 (s, 1H).
MS (ESI) m/z: 310 [M+H]⁺.

### Reference Example E012: production of (E)-2-((tertbutyldimethylsilyl)oxy)-3-((2-(4-(5-(methylamino)pyrazin-2-yl)but-1-en-3-yn-1-yl)thiazolo[5,4-b]pyridin-5-yl)oxy)propyl 4-methylbenzenesulfonate

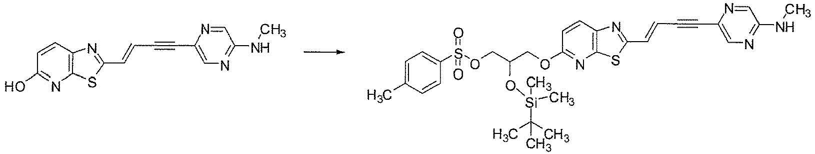

To a solution of unpurified (E)-2-(4-(5-(methylamino)pyrazin-2-yl)but-1-en-3-yn-1-yl)thiazolo[5,4-b]pyridin-5-ol (1.81 g) produced in Reference Example E011, 2-((tert-butyldimethylsilyl)oxy)-3-hydroxypropyl 4-methylbenzenesulfonate (2.63 g) produced by a production method in Reference Example 15 of WO 2022/45093, and triphenylphosphine (3.06 g) in THF (29 ml), was added diisopropyl azodicarboxylate (40% toluene solution, 6.14 ml) at 0°C. The mixture was stirred at 0°C for 5 hours. Water and ethyl acetate were added to the mixture, and the aqueous layer was extracted with ethyl acetate. The organic layers were dried over anhydrous magnesium sulfate and filtered. The filtrate was concentrated under reduced pressure. The residue thus obtained was purified by column chromatography (silica gel, 0%-50% ethyl acetate/n-heptane) to obtain the title compound (3.82 g).
MS (ESI) m/z: 652 [M+H]⁺.

### Reference Example E013: production of (E)-2-hydroxy-3-((2-(4-(5-(methylamino)pyrazin-2-yl)but-1-en-3-yn-1-yl)thiazolo[5,4-b]pyridin-5-yl)oxy)propyl 4-methylbenzenesulfonate

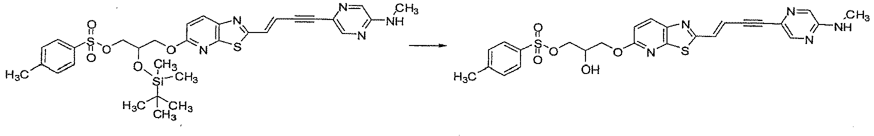

To a solution of (E)-2-((tert-butyldimethylsilyl)oxy)-3-((2-(4-(5-(methylamino)pyrazin-2-yl)but-1-en-3-yn-1-yl)thiazolo[5,4-b]pyridin-5-yl)oxy)propyl 4-methylbenzenesulfonate (3.42 g) produced in Reference Example E012 in THF (50 ml), were added acetic acid (6.01 ml) and tetra-n-butylammonium fluoride (1M THF solution, 26.2 ml) at 0°C. The mixture was stirred at room temperature for 18 hours and purified by column chromatography (ODS gel, 0% to 80% acetonitrile (containing 0.1% formic acid)/water (containing 0.1% formic acid)) to obtain the title compound (269 mg).
¹H-NMR (400 MHz, CDCl₃) δ (ppm): 2.42 (s, 3H), 3.04 (d, J=3.2 Hz, 3H), 4.12-4.30 (m, 3H), 4.46 (d, J=5.0 Hz, 2H), 5.10 (brs, 1H), 6.75-6.86 (m, 2H), 7.19 (d, J=15.9 Hz, 1H), 7.32 (d, J=8.2 Hz, 2H), 7.80 (d, J=8.6 Hz, 2H), 7.92 (d, J=1.8 Hz, 1H), 8.09 (d, J = 8.6 Hz, 1H), 8.22 (d, J=1.4 Hz, 1H).
MS (ESI) m/z: 538 [M+H]⁺.

### Reference Example E014: production of 2-(bromomethyl)-5-chlorothiazolo[5,4-b]pyridine and 5-chloro-2-(dibromomethyl)thiazolo[5,4-b]pyridine

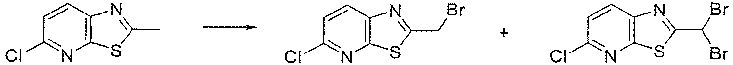

To a solution of 5-chloro-2-methylthiazolo[5,4-b]pyridine (CAS: 109202-21-3, 3 g) produced in Reference Example E006 in alpha, alpha, alpha-trifluorotoluene (108 ml), were added N-bromosuccinimide (4.34 g) and 2,2'-azobis(isobutyronitrile) (534 mg) at room temperature. The mixture was stirred at 95°C for 2 hours. To the mixture was added 2,2'-azobis(isobutyronitrile) (213 mg). The mixture was stirred at 95°C for 2 hours. Water and ethyl acetate were added to the mixture, and the aqueous layer was extracted with ethyl acetate. The organic layers were dried over anhydrous sodium sulfate and filtered. The filtrate was distilled under reduced pressure. The residue thus obtained was purified by column chromatography (silica gel, 0%-10% ethyl acetate/n-heptane) to obtain 2-(bromomethyl)-5-chlorothiazolo[5,4-b]pyridine (534 mg) and 5-chloro-2-(dibromomethyl)thiazolo[5,4-b]pyridine (2.88 g) separately. 2-(bromomethyl)-5-chlorothiazolo[5,4-b]pyridine:
¹H-NMR (400 MHz, CDCl₃) δ (ppm): 4.76 (s, 2H), 7.46 (d, J=8.6 Hz, 1H), 8.18 (d, J=8.6 Hz, 1H).
MS (ESI) m/z: 263, 265 [M+H]⁺.
5-chloro-2-(dibromomethyl)thiazolo[5,4-b]pyridine:
   ¹H-NMR (400 MHz, CDCl₃) δ (ppm): 6.86 (s, 1H), 7.49 (d, J=8.6 Hz, 1H), 8.19 (d, J=8.6 Hz, 1H).
   MS (ESI) m/z: 343, 345 [M+H]⁺.

### Reference Example E015: production 2 of 2-(bromomethyl)-5-chlorothiazolo[5,4-b]pyridine

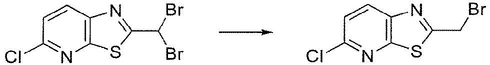

To a solution of 5-chloro-2-(dibromomethyl)thiazolo[5,4-b]pyridine (2.33 g) produced in Reference Example E014 in THF (14 ml), were added diisopropyl ethylamine (1.66 ml) and diethyl phosphite (1.58 ml) at 0°C. The mixture was stirred at 0°C for 1 hour. To the mixture was added diethyl phosphite (0.526 ml) at room temperature. The mixture was stirred for 30 minutes. Ethyl acetate, water, and a saturated ammonium chloride solution were added to the mixture, and the aqueous layer was extracted with ethyl acetate. The organic layer was washed with water and saturated brine, filtered through a small amount of silica gel, dried over anhydrous magnesium sulfate, and filtered. The filtrate was distilled under reduced pressure. The residue thus obtained was purified by column chromatography (silica gel, 3%-13% ethyl acetate/n-heptane) to obtain the title compound (1.58 g).
¹H-NMR (400 MHz, CDCl₃) δ (ppm): 4.76 (s, 2H), 7.46 (d, J=8.6 Hz, 1H), 8.17 (d, J=8.6 Hz, 1H).
MS (ESI) m/z: 263, 265 [M+H]⁺.

### Example 5: production 1 of (E)-1-fluoro-3-((2-(4-(5-(methylamino)pyrazin-2-yl)but-1-en-3-yn-1-yl)thiazolo[5,4-b]pyridin-5-yl)oxy)propan-2-ol (compound (III); hereinafter also referred to as "SPAL-E-17")

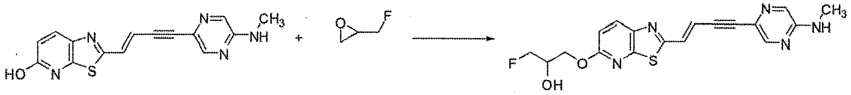

To a mixture of unpurified (E)-2-(4-(5-(methylamino)pyrazin-2-yl)but-1-en-3-yn-1-yl)thiazolo[5,4-b]pyridin-5-ol (32.8 mg) produced in Reference Example E011 and potassium carbonate (14.2 mg) in DMF (500 µl), was added 2-(fluoromethyl)oxirane (CAS: 503-09-3, 12.8 µl) at room temperature. The mixture was stirred at 80°C for 2 hours. After the mixture had been cooled to room temperature, a saturated ammonium chloride solution and ethyl acetate were added thereto, and the aqueous layer was extracted with ethyl acetate. The organic layers were combined, dried over anhydrous magnesium sulfate, and filtered. The filtrate was distilled under reduced pressure. The residue thus obtained was purified by column chromatography (ODS gel, 15% to 50% acetonitrile (containing 0.1% formic acid)/water (containing 0.1% formic acid)) to obtain the title compound (2.6 mg). ¹H-NMR (400 MHz, CDCl₃) δ (ppm): 3.03 (d, J=5.4 Hz, 3H), 3.85 (d, J=5.0 Hz, 1H), 4.23-4.35 (m, 1H), 4.48-4.58 (m, 3H), 4.59-4.68 (m, 1H), 4.90 (brd, J=5.0 Hz, 1H), 6.78 (d, J=15.9 Hz, 1H), 6.90 (d, J=9.1 Hz, 1H), 7.18 (d, J=15.9 Hz, 1H), 7.89 (d, J=1.4 Hz, 1H), 8.10 (d, J=9.1 Hz, 1H), 8.21 (d, J=1.4 Hz, 1H). MS (ESI) m/z: 386 [M+H]⁺.

### Example 6: production of (E)-3-((2-(4-(5-(methylamino)pyrazin-2-yl)but-1-en-3-yn-1-yl)thiazolo[5,4-b]pyridin-5-yl)oxy)-2-((tetrahydro-2H-pyran-2-yl)oxy)propyl 4-methylbenzenesulfonate

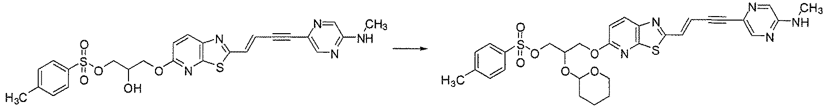

To a solution of (E)-2-hydroxy-3-((2-(4-(5-(methylamino)pyrazin-2-yl)but-1-en-3-yn-1-yl)thiazolo[5,4-b]pyridin-5-yl)oxy)propyl 4-methylbenzenesulfonate (269 mg) produced in Reference Example E013 in dichloromethane (5 ml), were added 3,4-dihydro-2H-pyrane (0.091 ml) and p-toluenesulfonic acid monohydrate (47.7 mg) at 0°C. The mixture was stirred at room temperature for 1 hour, followed by addition of 3,4-dihydro-2H-pyrane (0.046 ml) at 0°C. After the mixture had been stirred at room temperature for 30 minutes, a saturated sodium hydrogencarbonate solution and ethyl acetate were added thereto, and the aqueous layer was extracted with ethyl acetate. The organic layer was dried over anhydrous magnesium sulfate and filtered. The filtrate was concentrated under reduced pressure. The residue thus obtained was purified by column chromatography (silica gel, 0%-100% ethyl acetate/n-heptane) to obtain the title compound (57.5 mg).
MS (ESI) m/z: 622 [M+H]⁺.

### Example 7: production 2 of (E)-1-fluoro-3-((2-(4-(5-(methylamino)pyrazin-2-yl)but-1-en-3-yn-1-yl)thiazolo[5,4-b]pyridin-5-yl)oxy)propan-2-ol (compound III)

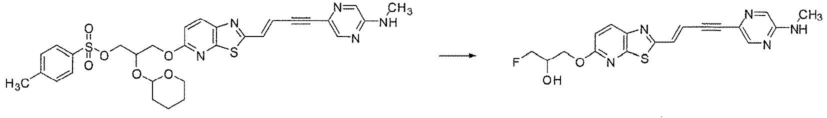

To a solution of potassium fluoride (2.8 mg) and tetrabutylammonium iodide (1.49 mg) in water (0.50 ml), was added a solution of 4,7, 13, 16,21,24-hexaoxa-1,10-diazabicyclo[8.8.8]hexacosane (Kryptofix (registered trademark) 222) (18.2 mg) in acetonitrile (0.50 ml) at room temperature. The mixture was concentrated at 40°C under reduced pressure. To the residue thus obtained was added acetonitrile (1.0 ml). The mixture was concentrated at 40°C under reduced pressure. To the residue thus obtained was added acetonitrile (1.0 ml) again. The mixture was concentrated at 40°C under reduced pressure. To the residue thus obtained was added toluene (1.0 ml). The mixture was concentrated at 40°C under reduced pressure. To the residue thus obtained was added acetonitrile (1.0 ml). The mixture was concentrated at 40°C under reduced pressure. The residue thus obtained was dried at 100°C for 10 minutes under reduced pressure. To the residue thus obtained was added a solution of (E)-3-((2-(4-(5-(methylamino)pyrazin-2-yl)but-1-en-3-yn-1-yl)thiazolo[5,4-b]pyridin-5-yl)oxy)-2-((tetrahydro-2H-pyran-2-yl)oxy)propyl 4-methylbenzenesulfonate (5.0 mg) produced in Example 6 in DMSO (0.60 ml) at 60°C. The mixture was stirred at 60°C for 90 minutes. After the reaction solution had been cooled to room temperature, water (0.30 ml) and trifluoroacetic acid (0.70 ml) were added thereto. The mixture was stirred at room temperature for 5 minutes. The mixture was purified by column chromatography (ODS gel, 0% to 40% acetonitrile (containing 0.1% formic acid)/water (containing 0.1% formic acid)) to obtain the title compound (2.1 mg).
¹H-NMR (400 MHz, CDCl₃) δ (ppm): 3.03 (d, J=5.0 Hz, 3H), 4.24-4.34 (m, 2H), 4.46-4.58 (m, 3H), 4.63 (t, J=4.8 Hz, 1H), 5.03 (brd, J=4.1 Hz, 1H), 6.74-6.83 (m, 1H), 6.90 (d, J=9.1 Hz, 1H), 7.18 (d, J=15.9 Hz, 1H), 7.91 (s, 1H), 8.10 (d, J = 8.6 Hz, 1H), 8.21 (s, 1H).
MS (ESI) m/z: 386 [M+H]⁺.

### (Test Examples)

### [Optical imaging of human brain]

### (Dissected brain tissue)

Postmortem human brains were obtained in autopsies carried out with respect to a patient with dementia with Lewy bodies (DLB) and a patient with Alzheimer's disease (AD). In a cryostat (HM560, Carl Zeiss), frozen tissue from the DLB patient was sliced into sections each having a thickness of 20 µm. Brain tissue from the AD patient was fixed in 10% neutral buffered formalin, embedded in a paraffin block, and sliced into sections each having a thickness of 6 µm.

### (In vitro fluorescence microscope measurement)

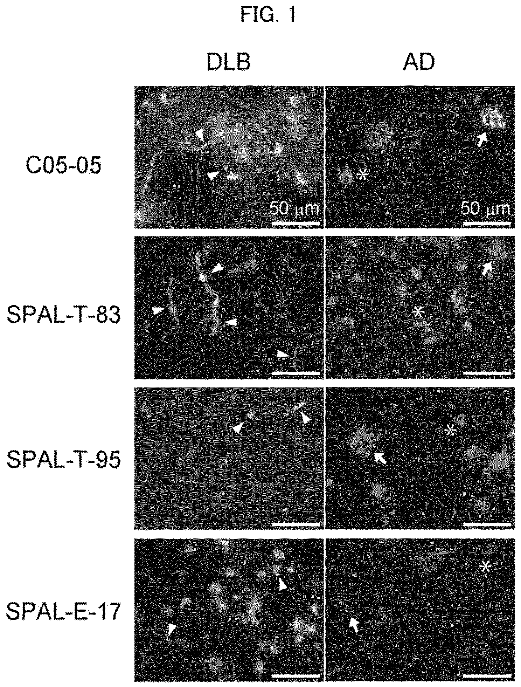

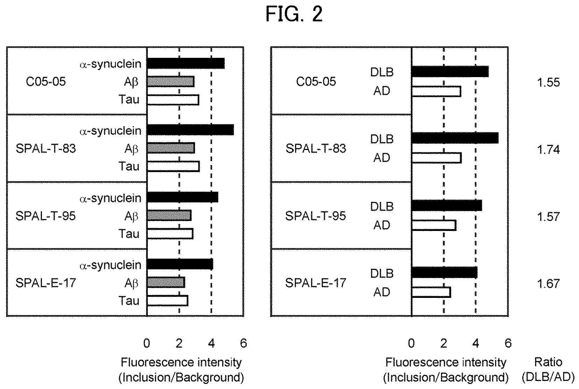

Postfixed fresh frozen sections of amygdala tissue obtained from the brain of the DLB patient and deparaffinized sections of formalin-fixed paraffin-embedded superior temporal gyrus tissue obtained from the brain of the AD patient were used. Each brain section and a 30 µmol/l test compound were incubated in a 50% ethanol solution at room temperature for 30 minutes. Subsequently, the sections were rinsed with 50% ethanol for 5 minutes and dipped into double-distilled water twice for 3 minutes each. After the section was mounted in a mounting medium (VECTASHIELD H-1000, Vector Laboratories), an image of a lesion-enriched area on the section was obtained with use of a fluorescence microscope (DM4000, Leica). Fluorescence images are shown in Fig. 1. In Fig. 1, each arrowhead indicates fluorescence from the compound binding to α-synuclein aggregates in the brain of the DLB patient, each arrow indicates fluorescence from the compound binding to amyloid-β aggregates in the brain of the AD patient, and an asterisk indicates fluorescence from the compound binding to tau aggregates in the brain of the AD patient. The fluorescence intensities of lesion-enriched areas and lesion-free areas (background) were quantified with use of analysis software (Image J). Results are shown in Fig. 2. Note that SPAL-T-83, SPAL-T-95, and SPAL-E-17 were each used as the test compound. Note also that ((E)-1-fluoro-3-(2-(4-(6-(methylamino)pyridin-3-yl)but-1-en-3-ynyl)benzo[d]thiazol-6-yloxy)propan-2-ol (C05-05) synthesized by the method disclosed in Synthetic Example 21 of Patent Literature 2 (International Publication No. WO 2020/174963) listed above was used as a control compound.

As shown in Figs. 1 and 2, it was found that SPAL-T-83, SPAL-T-95, and SPAL-E-17 each bound to α-synuclein aggregates formed in the brain of the DLB patient. It was also found that binding of each of SPAL-T-83, SPAL-T-95, and SPAL-E-17 to the α-synuclein aggregates was greater than binding of each of SPAL-T-83, SPAL-T-95, and SPAL-E-17 to tau aggregates or amyloid-β aggregates formed in the brain of the AD patient. Further, it was found that a ratio of binding of each of SPAL-T-83, SPAL-T-95, and SPAL-E-17 to the α-synuclein aggregates to binding of each of SPAL-T-83, SPAL-T-95, and SPAL-E-17 to tau aggregates or amyloid-β aggregates was higher than a ratio of binding of C05-05 to the α-synuclein aggregates to binding of C05-05 to tau aggregates or amyloid-β aggregates.

### [Optical imaging of mouse brain]

### (Preparation of α-synuclein fibril-inoculated mouse model)

When mouse α-synuclein is expressed in and extracted from E. coli as a recombinant protein and then incubated in vitro, insoluble α-synuclein aggregates are formed. By inoculating these α-synuclein aggregates into the striatum of a mouse, the α-synuclein aggregates propagate to surrounding areas via the neural circuitry, and an α-synuclein lesion is observed in the cerebral neocortex several months later (Masuda-Suzukake et al. Acta Neuropathol Commun 2, 88, 2014; Shimozawa et al. Acta Neuropathol Commun 5, 12, 2017). By extracting the brain of this mouse, preparing a section, and then analyzing the section by fluorescent staining, it is possible to examine whether the compound in accordance with an embodiment of the present invention binds to a lesion caused by phosphorylated α-synuclein.

### (Preparation of α-synuclein fibril-inoculated mouse model)

First, mouse α-synuclein was expressed in and extracted from E. coli as a recombinant protein, and then incubated in vitro to form insoluble α-synuclein aggregates. The α-synuclein aggregates were then inoculated into the striatum of a mouse. The α-synuclein aggregates propagated to surrounding areas via the neural circuitry, and α-synuclein lesions were observed in the cerebral neocortex several months later. Note that the inoculation of the α-synuclein aggregates into the striatum of the mouse was carried out in the following manner. First, hair on the head of a 9-week-old C57/BL/6 male mouse which had been anesthetized with 1.5% (v/v) isoflurane was removed, and the scalp was disinfected with isodine, followed by application of xylocaine and incision in the scalp to expose the skull. Then, a hole was drilled in the skull at a position of 0.05 mm posterior to the bregma and 2 mm lateral to the midline, and 3 µl of an α-synuclein fibril solution (mouse α-synuclein fibril (4 mg/ml) in saline) was injected into a position 2 mm ventral to the brain surface with use of a glass pipette. The scalp was then returned and sutured.

### (In vitro fluorescence microscope measurement)

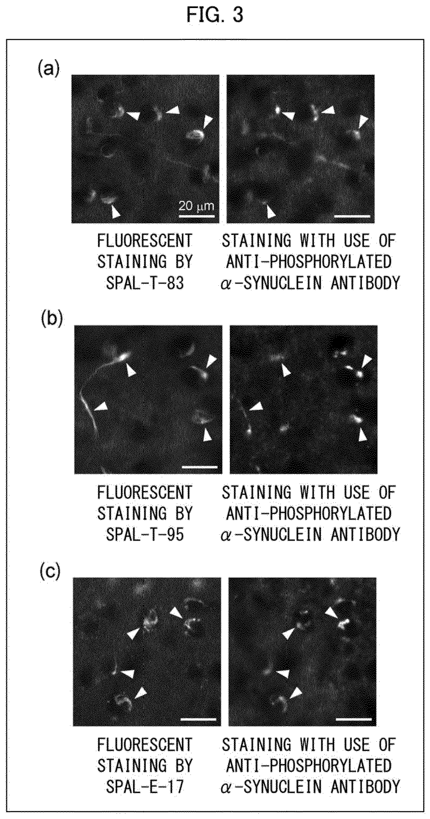

The brain of this α-synuclein fibril-inoculated mouse was extracted, and then sections were prepared. The sections were analyzed by fluorescent staining. Specifically, each brain section of the α-synuclein fibril-inoculated mouse and a 30 µmol/l test compound were incubated in a 20% ethanol solution at room temperature for 30 minutes. Subsequently, the sections were rinsed with 20% ethanol for 5 minutes and dipped into double-distilled water twice for 3 minutes each. After the section was mounted in a mounting medium (VECTASHIELD H-1000), an image of an α-synuclein aggregate-enriched area on the section was obtained with use of a fluorescence microscope (DM4000). The same section was washed with a phosphate buffer solution, and then treated in an autoclave for antigen retrieval. After the section was subjected to immunohistochemical staining with use of an anti-phosphorylated α-synuclein monoclonal antibody (pS129, abcam, ab59264) (1:1000) and then mounted in a mounting medium (VECTASHIELD H-1000), an image of the same area as above was obtained with use of a fluorescence microscope (DM4000). Results are shown in Fig. 3. (a) of Fig. 3 shows results obtained in relation to SPAL-T-83. (b) of Fig. 3 shows results obtained in relation to SPAL-T-95. (c) of Fig. 3 shows results obtained in relation to SPAL-E-17. In each of (a), (b), and (c) of Fig. 3, a right image shows a section subjected to staining with use of the anti-phosphorylated α-synuclein antibody, and a left image shows the section subjected to fluorescent staining by a corresponding one of SPAL-T-83, SPAL-T-95, and SPAL-E-17. In Fig. 3, each arrowhead indicates a lesion, caused by phosphorylated α-synuclein, in the brain of the α-synuclein fibril-inoculated mouse and fluorescence from the compound binding to the phosphorylated α-synuclein.

From the results shown in Fig. 3, it was indicated that SPAL-T-83, SPAL-T-95, and SPAL-E-17 bound to a lesion caused by phosphorylated α-synuclein.

### [In vivo two-photon laser scanning fluorescence microscopy]

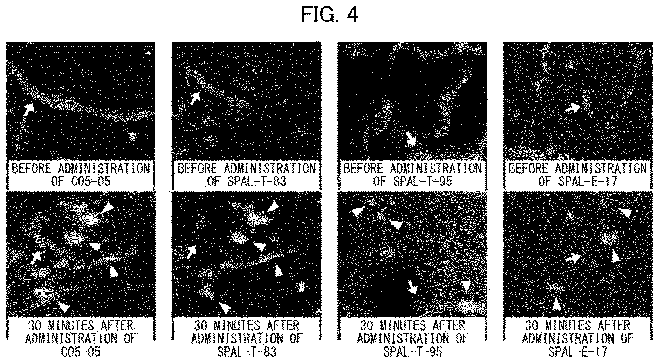

A model mouse was anesthetized with 1.5% (v/v) isoflurane 4 or more months after inoculation of an α-synuclein fibril solution. Then, a cranial window was placed on the model mouse in accordance with the Seylaz-Tomita method (Tomita et al. J Cereb Blood Flow Metab 25, 858-67, 2005). Two or more weeks after the placement of the cranial window, the mouse was fixed under a two-photon laser fluorescence microscope, 100 µl of physiological saline containing 5 mmol/l Sulforhodamine 101 was intraperitoneally administered to the mouse, and then intravital two-photon fluorescence imaging was carried out at an excitation wavelength of 900 nm. Thereafter, 50 µl of DMSO solution containing C05-05, SPAL-T-83, SPAL-T-95, or SPAL-E-17 at a 0.1% concentration was intraperitoneally administered to the mouse. Thirty minutes after the administration, intravital two-photon fluorescence imaging was carried out at an excitation wavelength of 900 nm. Detection wavelengths for C05-05, SPAL-T-83, SPAL-T-95, and SPAL-E-17 were set at 500 nm to 550 nm, and a detection wavelength for Sulforhodamine 101 was set at 573 nm to 648 nm. Results are shown in Fig. 4. In Fig. 4, each arrow indicates a blood vessel, and each arrowhead indicates fluorescence from the compound binding to an α-synuclein lesion.

From the results shown in Fig. 4, it was observed by the intravital two-photon laser fluorescence microscope that when each of SPAL-T-83, SPAL-T-95, and SPAL-E-17 was intravenously administered, each of these compounds, like C05-05, was delivered into the brain and further into neurons of the living body and bound to α-synuclein lesions. Therefore, it can be said that even in a case where lesions are at a low density or are small in total number and therefore it is difficult to detect the lesions by PET, it is possible to detect the lesions by biofluorescence imaging.

### Industrial Applicability

According to the present invention, it is possible to provide an α-synuclein aggregate binding agent having high binding selectivity with respect to α-synuclein aggregates. Furthermore, it is possible to provide a method for carrying out imaging with use of this α-synuclein aggregate binding agent. Moreover, it is possible to provide a novel compound which can be used for the α-synuclein aggregate binding agent or the other applications.

## Claims

1. A compound represented by the following formula (I), (II), or (III), a pharmaceutically acceptable salt thereof, or a solvate thereof.

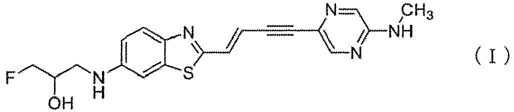

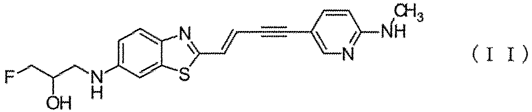

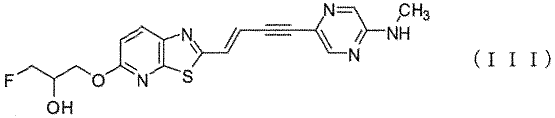

2. The compound, the pharmaceutically acceptable salt thereof, or the solvate thereof according to claim 1, wherein at least one atom in the compound represented by the formula (I), (II), or (III) is a radioisotope thereof.

3. An α-synuclein aggregate binding agent comprising a compound, a pharmaceutically acceptable salt, or a solvate thereof recited in claim 1.

4. An α-synuclein aggregate binding agent comprising a compound, a pharmaceutically acceptable salt, or a solvate thereof recited in claim 2.

5. A composition for optical imaging of α-synuclein aggregates, said composition comprising an α-synuclein aggregate binding agent recited in claim 3.

6. A composition for radiological imaging of α-synuclein aggregates, said composition comprising an α-synuclein aggregate binding agent recited in claim 4.

7. A method for carrying out optical imaging of α-synuclein aggregates in brain, said method comprising a step of detecting light which has a second wavelength and which is emitted from the brain of a living subject, after externally irradiating the brain with light which has a first wavelength, wherein an α-synuclein aggregate binding agent recited in claim 3 has been administered to the living subject, and the first wavelength and the second wavelength are different from each other.

8. A method for carrying out radiological imaging of α-synuclein aggregates in brain, said method comprising a step of detecting radioactivity which is emitted from the brain of a living subject to which an α-synuclein aggregate binding agent recited in claim 4 has been administered.

9. An intermediate for synthesizing a compound recited in claim 1 or 2, the intermediate being represented by the following formula (IV) or (V): 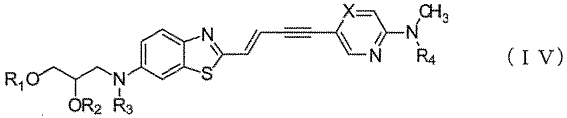 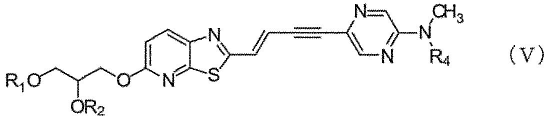 wherein, in the formulae (IV) and (V),
R₁ is a 4-nitrobenzenesulfonyl group, a p-toluenesulfonyl group, or a methanesulfonyl group,
R₂ is a tetrahydro-2H-pyran-2-yl group or a methoxymethyl group, and
R₄ is a hydrogen atom, a tert-butoxycarbonyl group, or a 2,4-dimethoxybenzyl group,
wherein, in the formula (IV),
X is a nitrogen atom (N) or an unsubstituted carbon atom (CH), and
R₃ is a hydrogen atom, a tert-butoxycarbonyl group, or a 2,4-dimethoxybenzyl group.

10. An intermediate for synthesizing a compound recited in claim 1 or 2, the intermediate being represented by the following formula (VI): 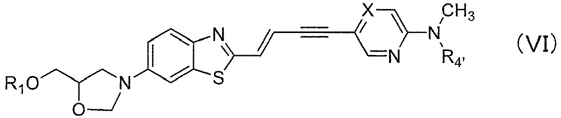 wherein:
R₁ is a 4-nitrobenzenesulfonyl group, a p-toluenesulfonyl group, or a methanesulfonyl group;
R₄ is a hydrogen atom or a tert-butoxycarbonyl group; and
X is a nitrogen atom (N) or an unsubstituted carbon atom (CH).
